# EUROPÄISCHE PATENTSCHRIFT

(11) **EP 1 005 553 B1**
(45) Veröffentlichungstag und Bekanntmachung des Hinweises auf die Patenterteilung: **09.05.2007**
(21) Anmeldenummer: 98942648.1
(22) Anmeldetag: 27.07.1998
(51) Int. Cl.: C12N 15/31, C12N 15/62

(54) **SEKRETION VON TRAGERGEBUNDENEN PROTEINEN IN DAS PERIPLASMA UND IN DEN EXTRAZELLULÄREN RAUM**
SECRETION OF CARRIER-BONDED PROTEINS INTO THE PERIPLASMA AND THE EXTRACELLULAR SPACE
SECRETION DE PROTEINES LIEES A DES SUPPORTS DANS LE CYTOPLASME ET DANS L'ESPACE EXTRACELLULAIRE

(30) Priorität: 30.07.1997 DE 19732829
(43) Veröffentlichungstag der Anmeldung: 07.06.2000
(73) Patentinhaber: Prof. Dr. Lubitz, Werner, 3420 Klosterneuburg/Kritzendorf (AT)
(72) Erfinder: LUBITZ, Werner, A-1080 Wien (AT); RESCH, Stephanie, D-81477 München (DE)
(74) Vertreter: Weiss, Wolfgang
(86) Internationale Anmeldenummer: PCT/EP1998/004723
(87) Internationale Veröffentlichungsnummer: WO 1999/006567

(56) Entgegenhaltungen:
- WO-A-95/19371
- KUEN B ET AL: "HETEROLOGOUS EXPRESSION AND SELF-ASSEMBLY OF THE S-LAYER PROTEIN SBSA OF BACILLUS STEAROTHERMOPHILUS IN ESCHERICHIA COLI" MOLECULAR MICROBIOLOGY, Bd. 19, Nr. 3, Februar 1996, Seiten 495-503, XP000675407
- KUEN B ET AL: "MOLECULAR CHARACTERIZATION OF THE BACILLUS STEAROTHERMOPHILUS PV72 S-LAYER GENE SBSB INDUCED BY OXIDATIVE STRESS" JOURNAL OF BACTERIOLOGY, Bd. 179, Nr. 5, März 1997, Seiten 1664-1670, XP000674432
- CLEMENT J -M ET AL: "Secretion of a bacterial protein by mammalian cells" JOURNAL OF BIOTECHNOLOGY, Bd. 43, Nr. 3, 15. Dezember 1995, Seite 169-181 XP004036873 in der Anmeldung erwähnt
- PEYRET J L ET AL: "CHARACTERIZATION OF THE CSPB GENE ENCODING PS2, AN ORDERED SURFACE-LAYER PROTEIN IN CORYNEBACTERIUM GLUTAMICUM" MOLECULAR MICROBIOLOGY, Bd. 9, Nr. 1, 1993, Seiten 97-109, XP000674434 in der Anmeldung erwähnt
- AUSUEBEL ET AL. (EDITORS): "Current protocols in molecular biology, Supplement 28, chapter 16.6.1-16.6.14" StartDateMarker 1994, EndDateMarker
- "NEW ENGLAND BIOLABS catalogue" 1996, NEW ENGLAND BIOLABS * Seite 164 - Seite 165 *
- NOMELLINI J F; LE K D; BINGLE W H; SMIT J: "Insertion of heterologous peptides within the surface-layer protein of Caulobacter crescentus" ABSTRACTS OF THE GENERAL MEETING OF THE AMERICAN SOCIETY FOR MICROBIOLOGY, Bd. 95, 1995, Seite 525,
- BAHL H; ET AL: "IV. MOLECULAR BIOLOGY OF S-LAYERS" FEMS MICROBIOLOGY REVIEWS, Bd. 20, 1997, Seiten 47-98,
- BINGLE W H; NOMELLINI J F; SMIT J: "LINKER MUTAGENESIS OF THE CAULOBACTER CRESCENTUS S-LAYER PROTEIN: TOWARD A DEFINITION OF AN N-TERMINAL ANCHORING REGION AND A C-TERMINAL SECRETION SIGNAL AND THE POTENTIAL FOR HETEROLOGOUS PROTEIN SECRETION" JOURNAL OF BACTERIOLOGY, Bd. 179, 1997, Seiten 601-611, XP002214899
- RIEDMANN EVA M; KYD JENNELLE M; SMITH ADAM M; GOMEZ-GALLEGO SARA; JALAVA KATRI; CRIPPS ALLAN W; LUBITZ WERNER: "Construction of recombinant S-layer proteins (rSbsA) and their expression in bacterial ghosts: A delivery system for the nontypeable Haemophilus influenzae antigen Omp26." FEMS IMMUNOLOGY AND MEDICAL MICROBIOLOGY, Bd. 37, Nr. 2-3, 15. Juli 2003 (2003-07-15), Seiten 185-192,

## Beschreibung

Die vorliegende Erfindung betrifft Verfahren zur Herstellung von trägergebundenen Proteinen, insbesondere von S-Layer-Proteinen und modifizierten S-Layer-Proteinen in pro karyontischen Wirtszellen.

Kristalline bakterielle Zelloberflächenlayer (S-Layer) bilden in vielen Eubakterien und den allermeisten Archaebakterien die äußerste Zellwandkomponente (Sleytr et al. (1988), Crystalline Bacterial Cell Surface Layers, Springer Verlag Berlin; Messner und Sleytr, Adv.Mikrob.Physiol.33 (1992), 213-275). Die meisten der gegenwärtig bekannten S-Layer-Proteine sind aus identischen Proteinen bzw. Glykoproteinen zusammengesetzt, die scheinbare Molekulargewichte im Bereich von 40 000 bis 220 000 aufweisen. Die Komponenten von S-Layern sind selbst-assemblierend und die meisten Gitter haben eine schräge (p2), quadratische (p4) oder hexagonale (p6) Symmetrie. Die Funktionen von bakteriellen S-Layern sind immer noch nicht vollständig bekannt, aber aufgrund ihrer Lokalisierung an der Zelloberfläche dürften die porösen kristallinen S-Layer hauptsächlich als Schutzhüllen, Molekularsiebe oder zur Förderung der Zelladhäsion und Oberflächenerkennung dienen.

Genetische Daten und Sequenzinformationen sind für verschiedene S-Layer-Gene aus Mikroorganismen bekannt. Eine Übersicht findet sich bei Peyret et al., Mol. Microbiol. 9 (1993), 97-109. Auf diese Daten wird ausdrücklich Bezug genommen. Die Sequenz des für das S-Layer-Protein von B.stearothermophilus PV72 kodierenden Gens sbsA und ein Verfahren zu dessen Klonierung sind bei Kuen et al. (Gene 145 (1994), 115-120) angegeben.

B.stearothermophilus PV72 ist ein gram-positives Bakterium, das mit einem hexagonal angeordneten S-Layer bedeckt ist. Die Hauptkomponente des S-Layer ist ein 128 kd-Protein, bei dem es sich um das häufigste Protein in der Zelle mit einem Anteil von ungefähr 1 5% bezüglich der gesamten Proteinbestandteile handelt. Es sind verschiedene Stämme von B.stearothermophilus charakterisiert worden, die hinsichtlich des Typs von S-Layer-Gitter, dem Molekulargewicht und der Glykosilierung der S-Layer-Komponenten unterschiedlich sind (Messner und Sleytr (1992), supra).

Die deutsche Patentanmeldung DE-OS 44 25 527 offenbart den Signalpeptid-kodierenden Abschnitt des S-Layer-Gens aus B.stearothermophilus und die davon abgeleitete Aminosäuresequenz. Die Spaltstelle zwischen dem Signalpeptid und dem reifen Protein befindet sich zwischen Position 30 und 31 der Aminosäuresequenz. Die Signalpeptid-kodierende Nukleinsäure kann operativ mit einer Protein-kodierenden Nukleinsäure verknüpft werden und zur rekombinanten Herstellung von Proteinen in einem Verfahren verwendet werden, bei dem man eine transformierte Wirtszelle bereitstellt, die Wirtszelle unter Bedingungen kultiviert, die zu einer Expression der Nukleinsäure und zu einer Erzeugung und Sekretion des davon kodierten Polypeptids führen, und das resultierende Polypeptid aus dem Kulturmedium gewinnt. Als Wirtszellen werden vorzugsweise prokaryontische Organismen, insbesondere gram-positive Organismen der Gattung Bacillus genannt.

In der internationalen Patentanmeldung PCT/EP97/00432 wird die rekombinante Herstellung von S-Layer-Proteinen und modifizierten S-Layer-Proteinen im Cytoplasma gram-negativer Wirtszellen vorgeschlagen. (vgl auch KUEN ET AL. MOLECULAR BIOLOGY 1995, 19(3), 495-503)

Überraschenderweise wurde nun festgestellt, daß die rekombinante Herstellung von S-Layer-Proteinen nicht nur im Cytoplasma gram-negativer prokaryontischer Wirtszellen möglich ist, sondern daß auch eine rekombinante Expression durchgeführt werden kann, die eine Sekretion in das Periplasma umfaßt.

Ein erster Aspekt der vorliegenden Erfindung ist somit ein Verfahren zur Herstellung der S-Layer Proteine, SbsA oder SbsB, dadurch gekennzeichnet, daß man
(a) eine gram-negative prokaryontische Wirtszelle bereitstellt, die transformiert ist mit einer für ein S-Layer-Protein kodierenden Nukleinsäure in operativer Verknüpfung, 5'-seitig von der Nukleinsäure, mit der MalE-Signalsequenz, die für ein Peptid kodiert, das eine Sekretion des S-Layer-Proteins in den periplasmatischen Raum der Wirtszelle bewirkt,
(b) die Wirtszelle unter solchen Bedingungen kultiviert, die zu einer Expression der Nukleinsäure und zu einer Erzeugung des davon kodierten Polypeptids führen und
(c) gegebenenfalls das resultierende Polypeptid aus dem periplasmatischen Raum der Wirtszelle gewinnt.

Überraschenderweise wurde festgestellt, daß eine Sekretion der S-Layer-Proteine SbsA oder SbsB, auch rekombinanter S-Layer-Proteine in den periplasmatischen Raum einer gram-negativen Wirtszelle möglich ist. Dabei bildet sich das S-Layer-Protein im Periplasma der Wirtszelle nicht in Form von ungeordneten Einschlußkörpern, sondern unerwarteterweise in Form von geordneten monomolekularen Schichten.

Vorzugsweise ermöglicht das erfindungsgemäße Verfahren die Expression von S-Layer-Genen, die aus B.stearothermophilus PV72 stammen, insbesondere die Expression der S-Layer Gene sbsA und sbsB. Daneben können aber auch S-Layer-Gene aus anderen Organismen (vgl. z.B. Peyret et al., (1993) supra) durch das erfindungsgemäße Verfahren exprimiert werden.

Die Nukleotidsequenz des für das reife SbsA-Protein kodierenden Gens ist in SEQ ID No. 1 von Position 91-3684 angegeben. Die zugehörige Aminosäuresequenz ist in SEQ ID No. 2 dargestellt. Die Nukleotidsequenz des für das reife SbsB-Protein kodierenden Gens ist in SEQ ID No. 5 von Position 94-2763 angegeben. Die zugehörige Aminosäuresequenz ist in SEQ ID No. 6 dargestellt.

In einer ersten bevorzugten Ausführungsform (sbsA) wird die Nukleinsäure, die für ein S-Layer-Protein kodiert, ausgewählt aus
(i) einer Nukleinsäure, welche die von Position 91 bis 3684 in SEQ ID No. 1 gezeigten Nukleotidsequenz umfaßt,
(ii) einer Nukleinsäure, welche eine der Nukleinsäure aus (i) im Rahmen der Degeneration des genetischen Codes entsprechende Nukleotidsequenz umfaßt, und
(iii) einer Nukleinsäure, welche eine mit den Nukleinsäuren aus (i) oder/und (ii) unter stringenten Bedingungen hybridisierende Nukleotidsequenz umfaßt.

In einer zweiten bevorzugten Ausführungsform (sbsB) wird die Nukleinsäure, die für ein S-Layer-Protein kodiert, ausgewählt aus
(i) einer Nukleinsäure, welche die von Position 94 bis 2763 in SEQ ID No. 5 gezeigte Nukleotidsequenz umfaßt,
(ii) einer Nukleinsäure, welche eine der Nukleinsäure aus (i) im Rahmen der Degeneration des genetischen Codes entsprechenden Nukleotidsequenz umfaßt und
(iii) einer Nukleinsäure, welche eine mit den Nukleinsäuren aus (i) oder/und (ii) unter stringenten Bedingungen hybridisierende Nukleotidsequenz umfaßt.

Unter dem Begriff "stringente Hybridisierung" im Sinne der vorliegenden Erfindung versteht man, daß eine Hybridisierung auch nach Waschen bei 55°C, vorzugsweise 60°C, in einem wäßrigen Niedrigsalz-Puffer (z.B. 0,2 x SSC) noch auftritt (s. auch Sambrook et al. (1989), Molecular Cloning. A Laboratory Manual).

Gemäß dem ersten Aspekt der Erfindung werden gram-negative prokaryontische Wirtszellen verwendet. Dabei wird überraschenderweise im Periplasma eine geordnete assemblierte S-Layer-Proteinstruktur erhalten. Vorzugsweise werden als Wirtszellen Enterobakterien, insbesondere E.coli, verwendet. Beispiele für geeignete E.coli Stämme sind DH5a (sup E44, Δ lac U169, hsdR17, recA1, endA1, gyr A96, thi-1, rel A1; Hanahan, J. Mol. Biol. 166 (1983), 557-580) und HB 2151 (K12, ara, Δ (lac-pro), thi/F', pro A + B + , laclqZAM15; Pharmacia Biotech).

Das erfindungsgemäße Verfahren kann auch zur Gewinnung rekombinanter S-Layer-Proteine eingesetzt werden. Hierzu verwendet man eine für das S-Layer-Protein kodierende Nukleinsäure, die eine oder mehrere Insertionen enthält, die für Peptid- oder Polypeptidsequenzen kodieren. Diese Insertionen können einerseits nur für Peptide mit wenigen Aminosäuren, z.B. 1-25 Aminosäuren, kodieren. Andererseits können die Insertionen auch für größere Polypeptide von z.B. bis zu 1000 Aminosäuren und vorzugsweise bis zu 500 Aminosäuren kodieren, ohne daß die Fähigkeit des S-Layer-Proteins zur Ausbildung einer korrekt gefalteten Struktur verlorengeht. Neben den Insertionen kann das rekombinante S-Layer-Protein auch Aminosäuresubstitutionen, insbesondere Substitutionen einzelner Aminosäuren im Bereich der Insertionsorte sowie gegebenenfalls Deletionen einzelner Aminosäuren oder kurzer Aminosäureabschnitte von bis zu 30 Aminosäuren aufweisen.

Als Insertionsstellen für Peptid- oder Polypeptid-kodierende Sequenzen in das sbsA-Gen bevorzugt sind Bereiche zwischen den Positionen 200-3600 der in SEQ ID N0.1 gezeigten Nukleotidsequenz. Besonders bevorzugte Insertionsstellen sind die Nrul-Schnittstelle an Position 585, die Pvull-Schnittstelle an Position 881, die SnaB-I-Schnittstelle an Position 920, die Pvull-Schnittstelle an Position 2507 und die Pvull-Schnittstelle an Position 2652 (PCT/EP 97/00 432). Weitere bevorzugte Insertionsstellen sind die Positionen 562, 1087, 1813, 1947, 2295, 2652, 3046, 3484 und 3594. Die jeweils angegebenen Positionen beziehen sich auf das erste Nukleotid der Insertion.

Als Insertionsstellen in das sbsB-Gen bevorzugt sind Bereiche zwischen den Positionen 200-2600 der in SEQ ID No. 5 gezeigten Nukleotidsequenz. Besonders bevorzugte Insertionsstellen sind die Positionen 410 (Codon 136), 484 (Codon 161/162) und 1583 (Codon 528/529) (PCT/EP 97/00432). Weitere bevorzugte Insertionsstellen sind die Positionen 598, 1012, 1435, 1808 und 2301, wobei sich die jeweils angegebene Position auf das erste Nukleotid der Insertion bezieht.

Die Peptid- oder Polypeptid-kodierenden Insertionen werden vorzugsweise ausgewählt aus Nukleotidsequenzen, die für Cysteinreste, Bereiche mit mehreren geladenen Aminosäuren, z.B. Arg. Lys, Asp oder Glu, oder Tyr-Resten, DNA-bindende Epitope, antigene, allergene oder immunogene Epitope, metallbindende Epitope, Streptavidin, Enzyme, Cytokine oder Antikörper-bindende Proteine kodieren.

Ein besonders bevorzugtes Beispiel für eine Insertion in die für das S-Laver-Protein kodierende Nukleinsäure ist eine für Streptavidin kodierende Nukleotidsequenz. Auf diese Weise können universelle Trägermoleküle erhalten werden, die zur Ankopplung von biotinylierten Reagenzien an das integrierte Streptavidin des rekombinanten S-Layerproteins und zum Nachweis in immunologischen oder Hybridisierungstestverfahren geeignet sind.

Ein weiteres bevorzugtes Beispiel für Insertionen sind antigene, allergene oder immunogene Epitope, z.B. Epitope aus pathogenen Mikroorganismen, wie etwa Bakterien, Pilzen, Parasiten etc. und Viren, oder Epitope aus Pflanzen oder Epitope gegen körpereigene Substanzen, z.B. Cytokine, sowie gegen Toxine, insbesondere Endotoxine. Besonders bevorzugte Beispiele für immunogene Epitope sind Epitope aus Viren, z.B. aus Herpesviren, wie etwa Herpesvirus 1 , z.B. Glykoprotein Δ, Herpesvirus 6 oder Pseudorabiesvirus (Lomniczi et al., J. Virol. 49 (1984), 970-979), insbesondere Epitope aus den Genen gB, gC oder/und gD, Epitope aus Maul- und Klauenseuchevirus (FMDV), insbesondere Epitope aus den Genabschnitten, die für VP1, VP2 oder/und VP3 kodieren, Epitope aus Flaviviren oder Epitope aus Filoviren wie etwa Ebola-, Marburg- oder Lassavirus. Die immunogenen Epitope können so ausgewählt werden, daß sie die Erzeugung einer Antikörpervermittelten Immunreaktion fördern oder/und die Erzeugung einer zellulären Immunreaktion, z.B. durch Stimulation von T-Zellen, fördern. Beispiele für geeignete allergene Epitope sind Birkenpollenallergene, z.B. Bet v I (Ebner et al., J. Immunol. 150 (1993) 1047-1054). Weiterhin besonders bevorzugt sind antigene Epitope, die in der Lage sind, aus Serum oder anderen Körperflüssigkeiten körpereigene oder körperfremde Substanzen wie etwa Cytokine oder Toxine zu binden und herauszufiltrieren. Derartige Epitope können Bestandteile von Cytokin- oder Toxinrezeptoren oder von Antikörpern gegen Cytokine oder Toxine umfassen.

Modifizierte S-Layer-Proteine, die immunogene oder/und antigene Epitope mit Glykosilierungsstellen aufweisen, werden vorzugsweise in eukaryontischen Wirtszellen hergestellt, in denen eine Glykosilierung möglich ist. Dabei können auch die natürlichen S-Layer-Sequenzen glykosiliert werden. Beispiele für potentielle N-Glykosilierungsstellen im S-Layer-Gen sbsA sind die Aminosäurepositionen 26, 285, 343, 384, 387, 388, 418, 421, 483, 653, 675, 902, 924, 1048, 1058, 1118, 1154 und 1161. Im sbsB-Gen kann eine potentielle N-Glykosilierung an den Positionen 155, 184, 213, 302, 303, 400, 463, 606, 755 und 915 stattfinden. Weitere mögliche Modifikationen des sbsA-Gens umfassen Amidierung, Phosphorylierung durch Caseinkinase II, N-Myristoylierung und Phosphorylierung durch Proteinkinase C. Weitere mögliche Modifikationen des sbsB Gens umfassen Phosphorylierung durch cAMP- und cGMP-abhängige Proteinkinase, Phosphorylierung durch Caseinkinase II, N-Myristoylierung, Phosphorylierung durch Proteinkinase C und Anlagerung an einen Fibronectin Rezeptor (über Sequenz RGD).

Andererseits können die Insertionen auch für Enzyme kodieren. Bevorzugte Beispiele sind Enzyme zur Synthese von Polyhydroxybuttersäure, z.B. PHB-Synthase. Durch Einbau von PHB-Synthase in den S-Layer kann bei Zufuhr des Substrats Hydroxybuttersäure unter geeigneten Bedingungen eine molekulare Spinndüse entstehen. Ein weiteres bevorzugtes Beispiel für ein Enzym ist bakterielle Luciferase. Hier kann bei Zufuhr des Enzymsubstrates, eines Aldehyds, sowie FMNH₂ (reduziertes Flavinmononukleotid), und in Anwesenheit von O₂ ein molekularer Laser erhalten werden.

Ebenfalls bevorzugt sind Insertionen, die für Cytokine, wie etwa Interleukine, Interferone oder Tumornekrosefaktoren kodieren. Diese Moleküle können beispielsweise in Kombination mit immunogenen Epitopen zur Herstellung von Vakzinen verwendet werden.

Schließlich sind auch Insertionen bevorzugt, die für Antikörper-bindende Proteine, wie etwa Protein-A oder Protein-G oder für DNA- oder/und metallbindende Epitope, wie etwa Leucin-Zipper, Zinkfinger etc. kodieren.

So wird durch die vorliegende Erfindung erstmals eine gram-negative prokaryontische Zelle bereitgestellt, die im Periplasma immobilisierte rekombinante Polypeptide in nativer Form, z. B. aktive Enzyme enthält. Pro mm² rekombinanten S-Layer können auf diese Weise 50.000 - 200.000, z.B. ca. 100.000 rekombinante Moleküle immobilisiert werden. Pro kg rekombinante E.coli Zellen können bis zu 3.000 m² S-Layer erhalten werden.

Darüber hinaus sind insbesondere für die Sekretion ins Periplasma rekombinante S-Layerproteine bevorzugt, in die Cysteinreste eingebaut wurden. Durch Auswahl der Insertionspositionen kann ein kovalentes Crosslinking der S-Layer im Periplasma erreicht werden oder/und bei Insertion an nicht zum Crosslinking geeignete Positionen können Andockstellen für Polypeptide, z.B. für Enzyme, bereitgestellt werden, die über eine freie SH-Gruppe kovalent mit dem S-Layer verknüpft werden können. Besonders bevorzugt eignen sich hierzu rekombinante Polypeptide, in die durch gentechnische Methoden ein zusätzlicher Cysteinrest, vorzugsweise am N- oder am C-Terminus oder an einer oberflächenlokalisierten Domäne eingeführt wurde und die durch Auswahl eines geeigneten Expressionssystems ebenfalls ins Periplasma der rekombinanten Wirtszelle sekretiert werden.

Bei dem erfindungsgemäßen Verfahren wird die für das S-Layer-Protein kodierende Nukleinsäure in operativer Verknüpfung mit einer für ein MalE Signalpeptid kodierenden Nukleinsäure verwendet, d.h. 5'-seitig von der S-Layer-Protein-kodierenden Nukleinsäure ist die Signalpeptid-kodierende Nukleinsäure angeordnet.

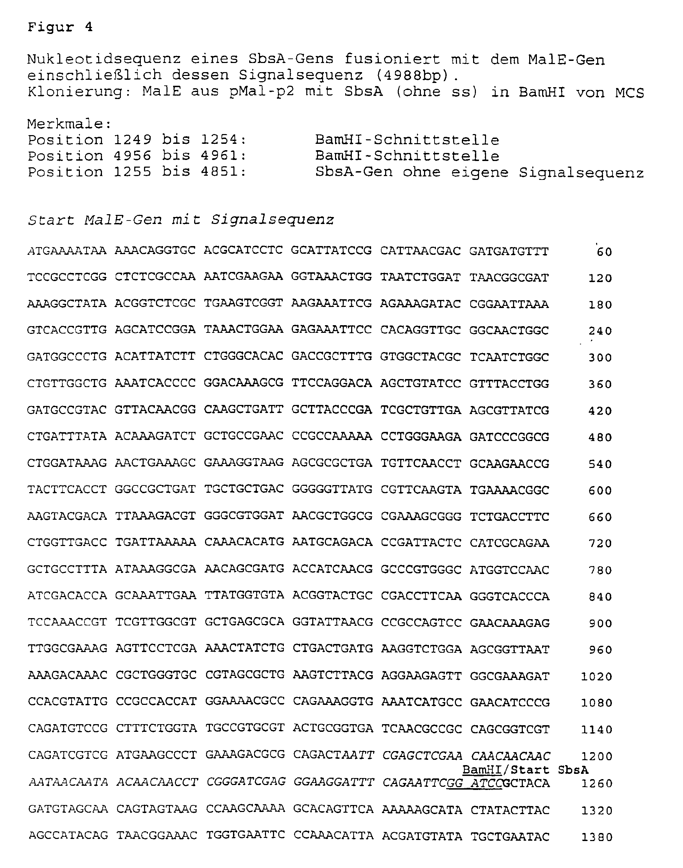

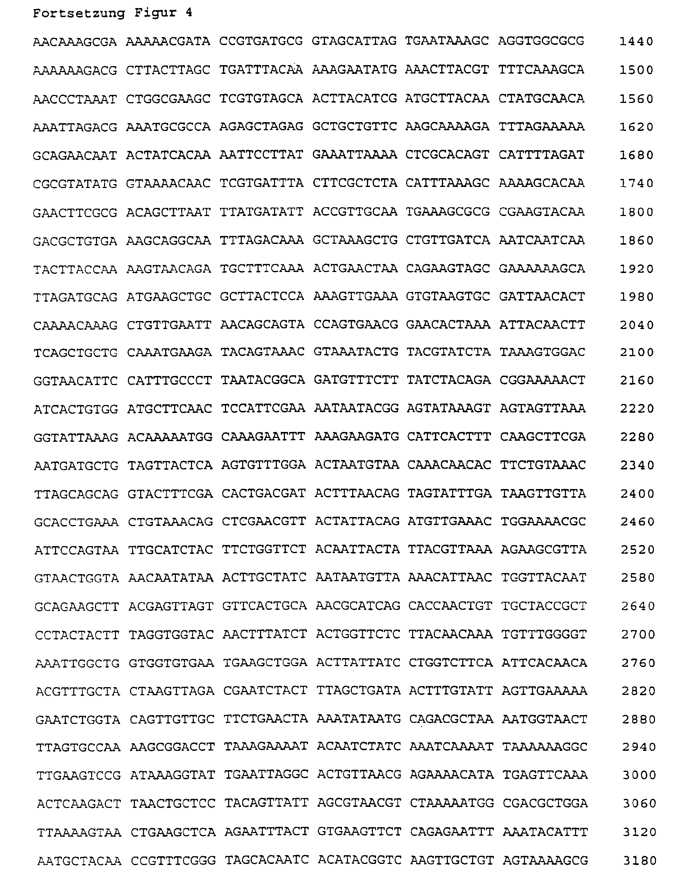

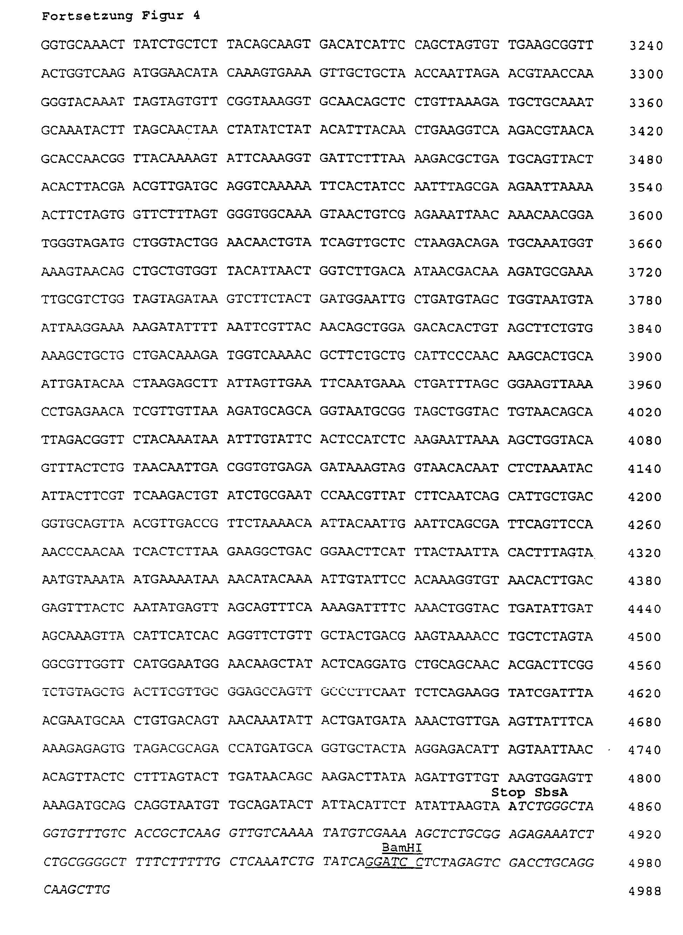

Bei einer Sekretion ins Periplasma gram-negativer prokaryontischer Zellen kann die für das Signalpeptid kodierende Nukleinsäure (a) den Signalpeptid-kodierenden Abschnitt der in SEQ ID No. 7 bzw. Fig. 4 dargestellten Nukleotidsequenz, (b) eine der Sequenz aus (a) im Rahmen der Degeneration des genetischen Codes entsprechende Nukleotidsequenz oder/und (c) eine zu den Sequenzen aus (a) oder/und (b) mindestens 80% und insbesondere mindestens 90% homologe Nukleotidsequenz umfassen. Andere Sequenzen, die eine Sekretion ins Periplasma bewirken, sind beispielsweise bei Blondel und Bedouelle (Eur. J. Biochem 193 (1990), 325-330; Adip-Conquy et al. (Protein Eng. 8 (1995), 859-863); Weller et al (Eur. J. Biochem. 236 (1996), 34-39) und Dubreuil et al. (FEMS Immunol. Med. Microbiol. 13 (1996), 317-323) beschrieben.

Fusionskonstrukte aus MalE und SbsA sowie aus MalE und SbsB sind in der vorliegenden Anmeldung beschrieben.

Neben dem für das Signalpeptid kodierenden Abschnitt kann die für das S-Layer-Protein kodierende DNA-Sequenz einen oder mehrere weitere Abschnitte enthalten, die für weitere Proteindomänen kodieren. Ein solcher Abschnitt kann vorzugsweise zwischen dem für das Signalpeptid kodierenden Abschnitt und dem für das S-Layer-Protein kodierenden Abschnitt angeordnet sein. Vorzugsweise kodiert dieser Abschnitt für ein sekretorisches Polypeptid aus gram-negativen bakteriellen oder eukaryontischen Organismen oder einen Teil davon. Ein bevorzugtes Beispiel für einen solchen Nukleinsäureabschnitt ist das malE Gen, welches das Maltose-Bindeprotein kodiert.

In einer bevorzugten Ausführungsform des erfindungsgemäßen Verfahrens können auch mehrere S-Layer-Gene in einer einzigen Wirtszelle exprimiert werden. Vorzugsweise werden zu diesem Zweck mindestens zwei S-Layer-Gene exprimiert, wobei eines davon für ein modifiziertes S-Layer-Protein und ein anderes für ein nicht modifiziertes S-Layer-Protein kodiert. Das nicht modifizierte S-Layer-Protein ist vorzugsweise in der Lage, eine mit dem modifizierten S-Layer-Protein kompatible S-Layer-Struktur auszubilden. Ein Beispiel für diese Ausführungsform des erfindungsgemäßen Verfahrens ist eine E.coli Zelle, welche mit zwei S-Layer-Genen transformiert ist, von denen eines ein natürliches sbsA- oder sbsB-Gen ist und das andere ein rekombinantes sbsA- oder sbsB-Gen ist.

Noch ein weiterer Gegenstand der vorliegenden Erfindung ist eine Nukleinsäure, die für ein gegebenenfalls heterologe Peptid- oder Polypeptidinsertionen enthaltendes S-Layer-Protein kodiert, ausgewählt aus SbsA oder SbsB, und in operativer Verknüpfung mit einer MalE-Signalsequenz ist, die für ein Peptid kodiert, das eine Sekretion in den periplasmatischen Raum einer gram-negativen prokaryontischen Wirtszelle bewirkt.

Vorzugsweise kodiert die Nukleinsäure für ein rekombinantes S-Layer-Protein wie zuvor angegeben.

Ein weiterer Gegenstand der vorliegenden Erfindung ist ein rekombinanter Vektor, der mindestens eine Kopie einer erfindungsgemäßen Nukleinsäure enthält. Der Vektor ist vorzugsweise in Prokaryonten oder/und in Eukaryonten replizierbar. Besonders bevorzugt ist der Vektor ein prokaryontisches oder ein eukaryontisches Plasmid. Weiterhin ist bevorzugt, daß der Vektor die erfindungsgemäße Nukleinsäure in operativer Verknüpfung mit einer in gram-negativen oder eukaryontischen Zellen aktiven Expressionskontrollsequenz ist. Besonders bevorzugt umfaßt die Expressionskontrollsequenz einen regulierbaren Promotor, Beispiele für geeignete prokaryontische Promotoren sind der tac-, lac-, trp- oder λ-Promotor.

Noch ein weiterer Gegenstand der vorliegenden Erfindung ist eine Wirtszelle, die mit einer Nukleinsäure oder einem rekombinanten Vektor gemäß vorliegender Erfindung transformiert ist. Vorzugsweise ist die Zelle eine gram-negative prokaryontische Zelle, z.B. eine E.coli-Zelle, Die erfindungsgemäße Zelle kann im Periplasma eine rekombinante S-Layerstruktur enthalten. Verfahren zur Transformation von Zellen mit Nukleinsäuren sind allgemeiner Stand der Technik (siehe Sambrook et al., supra) und brauchen daher nicht erläutert zu werden.

Aus rekombinanten S-Layer-Proteinmolekülen kann eine rekombinante S-Layer-Struktur assembliert werden, die als Untereinheit mindestens ein erfindungsgemäßes rekombinantes S-Layer-Protein enthält. Weiterhin ist bevorzugt, daß die erfindungsgemäße S-Layer-Struktur als "Verdünnungsmoleküle" auch nichtmodifizierte S-Layer-Proteine enthält. Die nichtmodifizierten S-Layer-Proteine liegen vorzugsweise in einem molaren Anteil von 10-99% bezüglich der gesamten S-Layer-Proteine vor.

Die erfindungsgemäße S-Layer-Struktur kann mehrere kovalent oder durch Affinitätsbindung miteinander verknüpfte Schichten umfassen. Kovalente Verknüpfungen können beispielsweise durch Insertionen von Cysteinresten und einer anschließenden Ausbildung von Cystinbrücken eingeführt werden. Verknüpfungen durch Affinitätsbindung umfassen beispielsweise Antikörper-Antigen-, Antikörper-Protein A- bzw. -Protein G- oder Streptavidin-Biotin-Wechselwirkungen.

S-Layer-Strukturen, die rekombinante S-Layer-Proteine enthalten, können gegebenenfalls auch in trägergebundener Form hergestellt werden. Hierzu kann die Reassemblierung der S-Layer-Struktur aus einzelnen Einheiten in Gegenwart eines Peptidoglycanträgers erfolgen, wobei beispielsweise Peptidoglycanschichten erzeugt werden, die auf einer oder auf beiden Seiten mit einer S-Layer-Struktur überzogen sind. Eine andere Möglichkeit zur Herstellung trägergebundener S-Layer-Strukturen besteht darin, eine S-Layer-Schicht an einer Grenzfläche zwischen zwei Medien, z.B. Wasser/Luft, zu erzeugen und diese Schicht auf einer Festphase, z.B. einer Filtermembran, zu immobilisieren (vgl. z.B. Pum und Sleytr (1994), Thin Solid Films 244, 882-886; Küpcü et al. (1995), Biochim. Biophys. Acta 1235, 263-269).

Die rekombinanten S-Layer-Proteine und S-Layer-Strukturen sind für eine Vielzahl von Anwendungen geeignet. Besonders bevorzugt ist die Verwendung als Vakzin oder Adjuvans, wobei man rekombinante S-Layer-Proteine verwendet, die immunogene Epitope von Pathogenen und/oder körpereigene immunstimulierende Polypeptide, wie etwa Cytokine, enthalten. Bei dieser Anwendung ist nicht unbedingt eine Reinigung der rekombinanten S-Layer-Proteine erforderlich. Stattdessen kann beispielsweise die Verwendung in Kombination mit einem Bakterienghost erfolgen, der ggf. in seinem periplasmatischen Raum, zusätzliche immunogene Epitope enthält.

Die Herstellung geeigneter "Bakterienghosts" ist beispielsweise in der internationalen Patentanmeldung PCT/EP91 /00967 beschrieben, auf die hiermit Bezug genommen wird. Dort werden modifizierte Bakterien offenbart, erhältlich durch Transformation eines gram-negativen Bakteriums mit dem Gen eines lytisch wirkenden Membranproteins aus Bakteriophagen, mit dem Gen eines lytisch wirkenden Toxin-Freisetzungsproteins oder mit Genen, die Teilsequenzen davon, die für lytische Proteine kodieren, enthalten. Kultivierung des Bakteriums. Expression dieses Lyse-Gens und Isolierung des resultierenden Bakterienghosts aus dem Kulturmedium.

An die Membran dieser Bakterien kann, wie im europäischen Patent 0 516 655 beschrieben, ein rekombinantes Protein gebunden sein, das durch Expression einer rekombinanten DNA in diesen gram-negativen Bakterien erhältlich ist. Diese rekombinante DNA umfaßt eine erste DNA-Sequenz, welche für eine hydrophobe, nicht lytisch wirkende membranintegrierende Proteindomäne, die eine α-helikale Struktur besitzt und aus 14-20 Aminosäuren besteht, die N- und C-terminal von je 2-30 beliebigen Aminosäuren flankiert sein können, kodiert. Mit dieser ersten DNA-Sequenz in operativer Verknüpfung befindet sich eine zweite DNA-Sequenz, die für ein gewünschtes rekombinantes Protein kodiert. Weiterhin enthält das gram-negative Bakterium eine dritte DNA-Sequenz, die unter einer von den ersten und zweiten DNA-Sequenzen getrennten Kontrolle steht und für ein lytisch wirkendes Membranprotein aus Bakteriophagen oder ein lytisch wirkendes Toxin-Freisetzungsprotein oder für deren lytisch wirkende Teile kodiert. Durch Expression und Lyse derartiger rekombinanter, gram-negativer Bakterien werden sog. "Bakterienghosts" erhalten, die eine intakte Oberflächenstruktur mit an die Oberfläche gebundenen immunogenen Epitopen enthalten.

Bei Kombination dieser Baktienghosts mit erfindungsgemäßen rekombinanten S-Layern können Vakzine und Adjuvantien erzeugt werden, die besonders vorteilhafte Eigenschaften aufweisen.

Eine weitere besonders bevorzugte Verwendung für rekombinante S-Layer-Proteine und S-Layer Strukturen ist die Verwendung als Enzymreaktor. Ein solcher Enzymreaktor kann beispielsweise von einer Zelle gebildet werden, die in ihrem Inneren eine erfindungsgemäße rekombinante S-Layer-Struktur enthält. Andererseits kann der Enzymreaktor auch aus isolierten und in vitro reassemblierten S-Layer-Strukturen oder Kombinationen verschiedender S-Layer-Strukturen gebildet werden.

Weiterhin wird die vorliegende Erfindung durch die nachfolgenden Beispiele und Figuren erläutert. Es zeigen:
- SEQ ID NO.1: die vollständige Nukleotidsequenz des kodierenden Abschnitts des S-Layer-Gens sbsA von B.stearothermophilus;
- SEQ ID NO.2: die davon abgeleitete Aminosäuresequenz;
- SEQ ID NO.3: die Nukleotidsequenz des Primers T5-X;
- SEQ ID NO.4: die Nukleotidsequenz des Primers E;
- SEQ ID NO.5: die vollständige Nukleotidsequenz des kodierenden Abschnitts des S-Layer-Gens sbsB von B.stearothermophilus;
- SEQ ID NO.6: die davon abgeleitete Aminosäuresequenz;
- SEQ ID NO.7: die Signalsequenz des malE Gens;
- SEQ ID NO.8: die Signalsequenz von Gen3 der Bakteriophagen fd;
- Fig.1: eine schematische Darstellung des zur Herstellung des rekombinanten Vektors pBK4 verwendeten sbsA PCR-Fragments;
- Fig.2: eine schematische Darstellung der Herstellung des das malE-sbsA-Fusionsgen enthaltenden Vektors pMAL-A (Beispiel 7),
- Fig.3: eine schematische Darstellung des Vektors pCant-A (Beispiel 8),
- Fig.4: die Nukleotidsequenz eines sbsA-Gens fusioniert mit dem malE-Gen einschließlich dessen Signalsequenz,
- Fig. 5: die Nukleotidsequenz eines sbsA-Gens fusioniert mit der Signalsequenz von Gen3 des Bakteriophage fd und

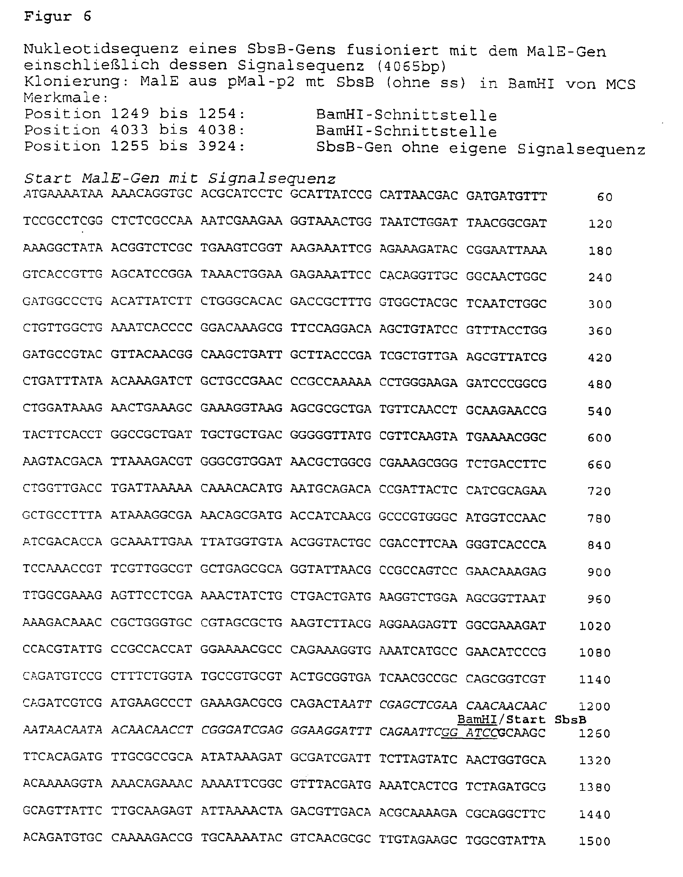

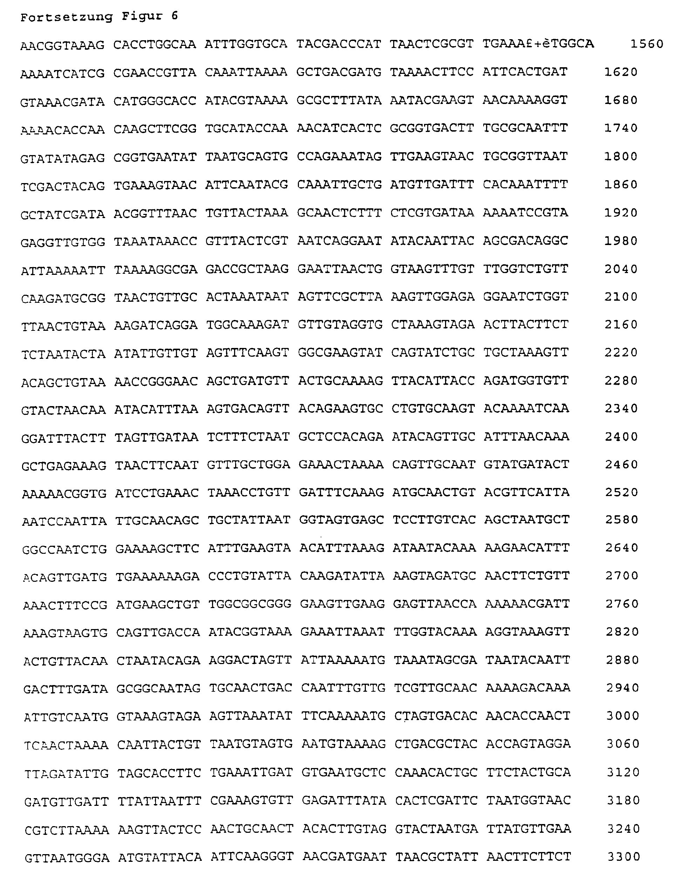

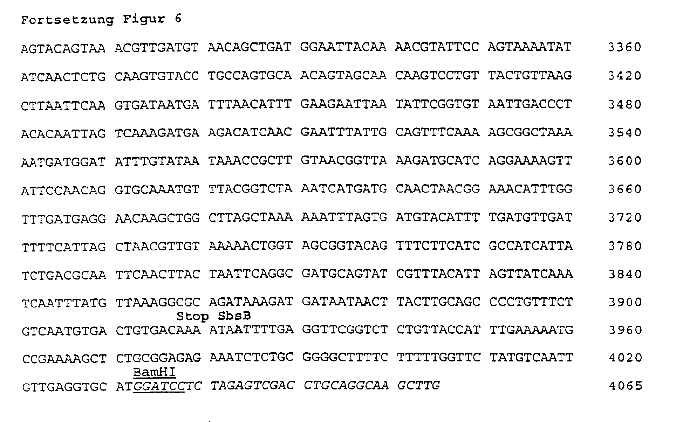

- Fig.6: die Nukleotidsequenz eines sbsB-Gens fusioniert mit dem malE-Gen einschließlich dessen Signalsequenz.

### BEISPIELE:

### 1. Bakterienstämme, Medien und Plasmide

Gram-positive Bakterien des Stammes Bacillus stearothermophilus PV72 wurden bei 58°C in SVIII-Medium (Bartelmus und Perschak, Z.Zuckerind.7 (1957), 276-281) kultiviert. E.coli Bakterien wurden in LB-Medium kultiviert (Sambrook et al., (1 989), supra). Zur Selektion von Transformanten wurde Ampicillin in einer Endkonzentration von 100 µg/ml dem Medium zugegeben. Das Plasmid pPLcAT10 (λpL, bla, colE1) (Stanssens et al., Gene 36 (1985), 211-223) wurde als Klonierungsvektor verwendet.

### 2. Manipulation von DNA-Fragmenten

Restriktionsanalyse von DNA, Agarosegelelektrophorese und Klonierung von DNA-Fragmenten wurden nach den bei Sambrook et al. (1989), supra, beschriebenen Standardmethoden durchgeführt.

Die Transformation von kompetenten Zellen erfolgte durch Elektroporation unter Verwendung eines Bio-Rad Genepulsers (Bio-Rad Laboratories, Richmond, Kalif., USA) nach Protokollen des Herstellers.

Plasmid-DNA wurde nach der Methode von Birnboim und Doly (Nucleic Acids Res.7 (1979), 1513-1523) isoliert. Chromosomale DNA wurde gemäß der bei Ausubel et al. (Current Protocols in Molecular Biology (1987), New York, John Wiley) beschriebenen Verfahren isoliert.

Restriktionsendonukleasen und andere Enzyme wurden von Boehringer Mannheim, New England Biolabs oder Stratagene bezogen und gemäß den Vorschriften der Hersteller eingesetzt.

### 3. DNA-Sequenzierung

Die Sequenzanalyse von DNA-Molekülen erfolgte nach der Didesoxykettenterminationsmethode von Sanger et al.. Die zur Sequenzierung des sbsA-Gens verwendeten Primer wurden auf Basis der bereits publizierten sbsA-Sequenz (Kuen et al., Gene 145 (1994), 115-120) konstruiert.

### 4 PCR-Amplifikation von sbsA

Die PCR-Amplifikation des sbsA-Gens erfolgte in einem Reaktionsvolumen von 100 µl, in dem 200 µM Desoxynukleotide, 1 U Pfu-Polymerase (Stratagene), 1x Pfu-Reaktionspuffer, jeweils 0.5 µM Oligonukleotidprimer und 100 ng genomischer DNA aus B.stearothermophilus als Matrize vorhanden waren. Die Amplifikation wurde über 30 Zyklen in einem Thermocycler (Biomed Thermocycler 60) durchgeführt. Jeder Zyklus bestand aus einem Denaturierungsschritt von 1,5 min bei 95°C, einem Annealingschritt von 1 min bei 56°C und 1 min bei 50°C sowie einem Extensionsschritt von 2 min bei 72°C.

Als Primer wurden der im Sequenzprotokoll als SEQ ID NO.3 angegebene Primer T5-X, der den 5'-Bereich von sbsA flankiert und eine Xbal-Stelle enthält, sowie der im Sequenzprotokoll in SEQ ID NO.4 gezeigte Primer E verwendet, der die 20 Nukleotide stromabwärts gelegene Region des Transkriptionsterminators der sbsA-Sequenz flankiert und eine BamHl-Stelle enthält.

Die PCR-amplifizierten Produkte wurden auf einem 0.8% Agarosegel elektrophoretisch aufgetrennt und zur Klonierung unter Verwendung des Systems von Gene Clean (BIO101 La Jolla, Kalif., USA) zur Klonierung gereinigt.

### 5. Klonierung des sbsA-Gens in den Vektor pPLcAT10

Das durch PCR gewonnene sbsA-Gen mit einer Länge von 3,79 kb wurde gereinigt und mit den Restriktionsendonukleasen Xbal und BamH1 gespalten. Das resultierende Xbal-BamHI-Fragment wurde in die entsprechenden Restriktionsstellen des Vektors pPLcAT10 kloniert, so daß das sbsA-Gen untertranskriptioneller Kontrolle des stromaufwärts gelegenen pL-Promotors war. Das ATG-Startkodon der sbsA-Sequenz wurde durch die Klonierungsprozedur rekonstruiert. Die klonierte sbsA-Sequenz enthielt die N-terminale Signalsequenz von sbsA und endete 20 nt nach dem Transkriptionsterminator. Nach Ligation der Vektor-DNA mit dem sbsA-Fragment wurde der E.coli-Stamm pop2135 (DSM10509) durch Elektrotransformation transformiert. Die resultierenden Klone wurden einer DNA-Restriktionsanalyse unterzogen. Ein positiver Klon wurde sequenziert, um die korrekten Sequenzübergänge an den 5'- und 3'-Enden zu verifizieren. Dieser Klon wurde als pBK4 bezeichnet.

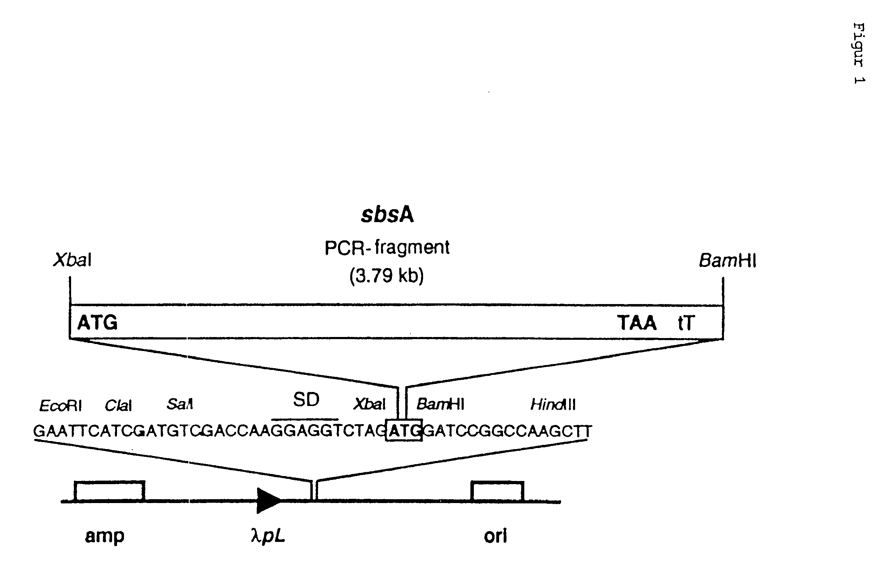

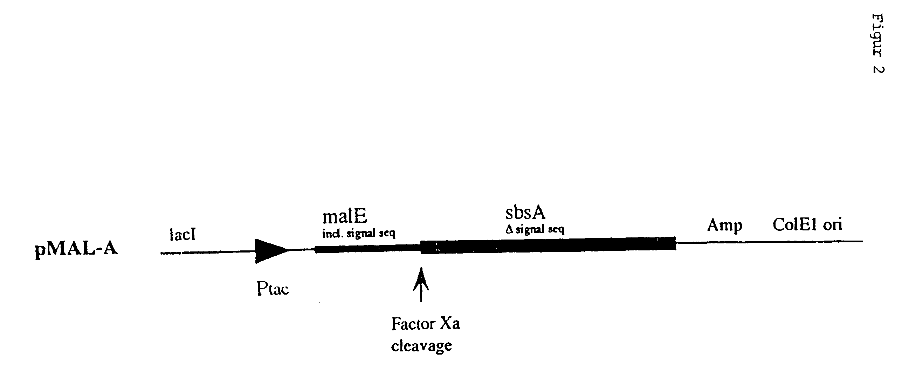

Eine schematische Darstellung des 3,79 kb Xbal sbsA-Fragments und seine Lokalisierung in der multiplen Klonierungsstelle des Plasmids pBK4 ist in Fig.1 dargestellt (Abkürzungen: tT: Transkriptionsterminator; ori: Ursprung der DNA-Replikation; amp: Ampicillinresistenzgen).

### 6. Rekombinante Expression des sbsA-Gens im Cytoplasma von E.coli (Vergleichsbeispiel)

E.coli pop2135/pBK4-Zellen wurden bei 28°C bis zum Erreichen einer optischen Dichte OD₆₀₀ von 0,3 kultiviert. Dann wurde die Expression von sbsA durch Erhöhung der Kultivierungstemperatur von 28°C auf 42°C induziert. 1,5 ml Aliquots wurden vor bzw. 1, 2, 3 und 5 Stunden nach Induktion der sbsA-Expression entnommen. Als Kontrollen wurden E.coli pop2135/pPLcAT10 (kultiviert unter den gleichen Bedingungen) und B.stearothermophilus PV72 verwendet.

Kulturüberstände und Zellextrakte aus allen Proben wurden auf die Expression des S-Layer-Proteins durch SDS-PAGE und Western-Immunoblotting untersucht.

Für den Western Blot wurden die Proteine auf eine Nitrozellulosemembran transferiert und mit einem polyklonalen Antiserum gegen SbsA aus Kaninchen inkubiert. Die Herstellung dieses Antiserums ist bei Egelseer et al. (J.Bacteriol.177 (1995), 1444-1451) beschrieben. Zum Nachweis gebundener SbsA-spezifischer Antikörper wurde ein Konjugat aus Ziegen-Anti-Kaninchen-IgG und alkalischer Phosphatase verwendet.

In cytoplasmatischen Extrakten aus mit pBK4 transformierten E.coli-Zellen wurde eine zusätzliche starke Proteinbande mit etwa dem gleichen Molekulargewicht wie das Wildtyp-SbsA-Protein gefunden.

Aus Überständen von mit pBK4 transformierten E.coli-Zellen konnte auch nach Induktion der sbsA-Gen-Expression kein SbsA-Protein nachgewiesen werden. Daraus ist ersichtlich, daß SbsA nicht in das umgebende Medium exportiert wird.

### 7. Sekretion des SbsA-Proteins ins Periplasma

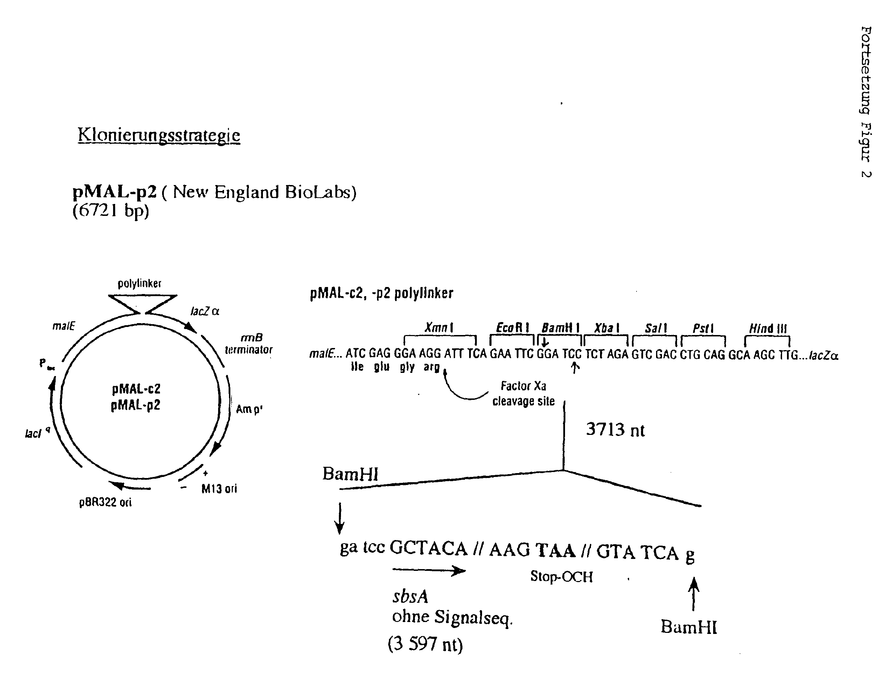

Das sbsA-Gen wurde ohne Signalsequenz und mit Stopkodon am 3'-Ende in den Polylinker des kommerziell erhältlichen Plasmids pMAL-P2 (New England Biolabs) kloniert (Fig. 2). Das resultierende Plasmid pMAL-A enthält unter Kontrolle des taq-Promotors ein Fusionsgen, umfassend das malE-Gen einschließlich dessen Signalsequenz sowie das sbsA-Gen ohne dessen Signalsequenz. Zwischen den beiden Domänen ist eine Faktor Xa Spaltungsstelle angeordnet.

Eine Analyse des Rohextrakts von mit pMAL-A transformierten E.coli DH5α-Zellen (Hanahan (1983) supra) zeigte die Expression eines MalE-SbsA Fusionspolypeptids mit einem Molekulargewicht von ca. 170 kDa in der durch eine kalte osmotische Schockprozedur (Neu und Heppel, J. Biol. Chem. 240 (1965); 3685-3692) erzeugten periplasmatischen Fraktion des Zellextrakts. Die Nukleotidsequenz des malE-sbsA-Fusionsgens ist in Fig. 4 dargestellt. Die malE-Signalsequenz ist in SEQ ID NO. 7 gezeigt.

### 8. Sekretion des SbsA-Proteins in den extrazellulären Raum (Vergleichsbeispiel)

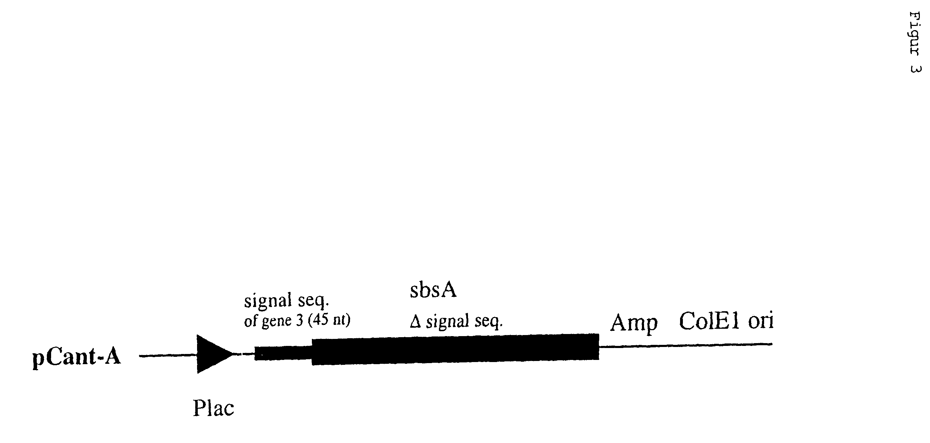

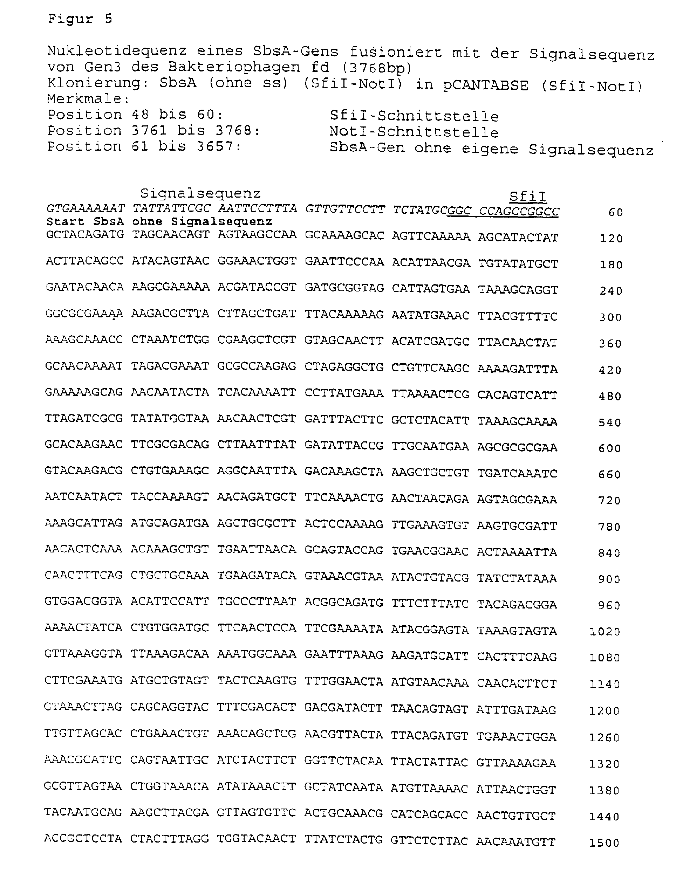

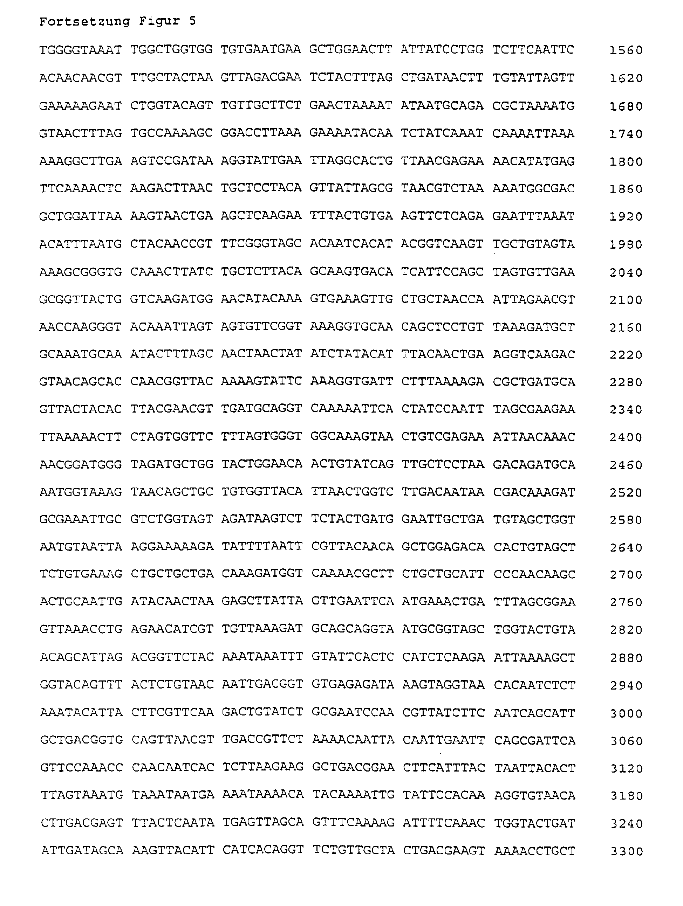

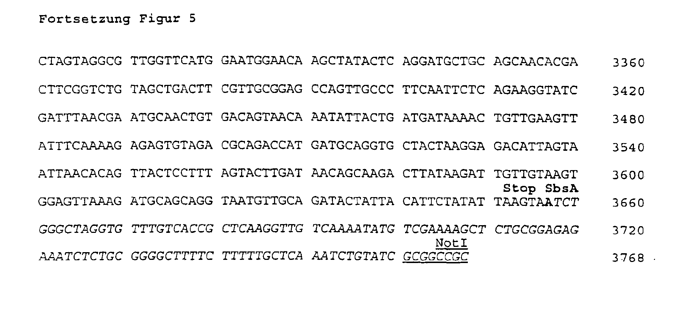

Das Plasmid pCant-A wurde hergestellt durch Klonierung des sbsA-Gens ohne eigene Signalsequenz und mit Stopcodon am 3'-Ende in das mit Sfil und Notl geschnittenen, kommerziell erhältlichen Plasmids pCANTAB5E (Pharmacia Biotech). Es enthält unter Kontrolle des lac-Promotors (Plac) die Signalsequenz von Gen 3 des Bakteriophagen fd (45 nt) fusioniert mit dem sbsA-Gen ohne dessen eigene Signalsequenz (Fig. 3). Die Nukleotidsequenz des Fusionsgens ist in Fig. 5 dargestellt. Die fdGen3 Signalsequenz ist in SEQ ID NO. 8 gezeigt.

Im Kulturüberstand von mit pCant-A transformierten E.coli HB2151 Zellen (Pharmacia Biotech) konnte das SbsA-Protein nachgewiesen werden.

### 9. Sekretion des SbsB-Proteins in das Periplasma und zum Vergleich in den extrazellulären Raum

Das sbsB-Gen wurde - wie in den Beispielen 7 und 8 beschrieben - ohne eigene Signalsequenz in die Plasmide pMAL-P2 und pCANTAB5E kloniert, wobei die Plasmide pMAL-B und pCant-B erhalten wurden.

In mit den Plasmiden pMAL-B und pCant-B transformierten E.coli-Zellen konnte eine Sekretion des SbsB-Proteins in das Periplasma und in den extraozellulären Raum gezeigt werden.

## Patentansprüche

1. Verfahren zur heterologen Herstellung der S-Layer-Proteine SbsA oder SbsB
**dadurch gekennzeichnet,**
**dass** man
(a) eine gram-negative prokaryontische Wirtszelle bereitstellt, die transformiert ist mit einer für ein S-Layer-Protein kodierenden Nukleinsäure in operativer Verknüpfung, 5'-seitig von der Nukleinsäure, mit der MalE-Signalsequenz, die für ein Peptid kodiert, das eine Sekretion des S-Layer-Proteins in den periplasmatischen Raum der Wirtszelle bewirkt,
(b) die Wirtszelle unter solchen Bedingungen kultiviert, die zu einer Expression der Nukleinsäure und zu einer Erzeugung des davon kodierten Polypeptids führen und
(c) gegebenenfalls das resultierende Polypeptid aus dem periplasmatischen Raum der Wirtszelle gewinnt.

2. Verfahren nach Anspruch 1,
**dadurch gekennzeichnet,**
**daß** die Nukleinsäure, die für ein S-Layer-Protein kodiert, ausgewählt wird aus
(i) einer Nukleinsäure, welche die von Position 91 bis 3684 in SEQ ID No.1 gezeigte Nukleotidsequenz umfaßt,
(ii) einer Nukleinsäure, welche die von Position 94 bis 2763 in SEQ ID No.5 gezeigte Nukleotidsequenz umfaßt,
(iii) einer Nukleinsäure, welche eine der Nukleinsäure aus (i) oder (ii) im Rahmen der Degeneration des genetischen Codes entsprechende Nukleotidsequenz umfaßt, und
(iv) einer Nukleinsäure, welche eine mit den Nukleinsäuren aus (i), (ii) oder/und (iii) unter stringenten Bedingungen hybridisierende Nukleotidsequenz umfaßt.

3. Verfahren nach einem der Ansprüche 1 bis 2,
**dadurch gekennzeichnet,**
**daß** die für das S-Layer-Protein kodierende Nukleinsäure eine oder mehrere Insertionen enthält, die für heterologe Peptid- oder Polypeptidsequenzen kodieren.

4. Verfahren nach Anspruch 3,
**dadurch gekennzeichnet,**
**daß** die Insertionsstelle
(a) an Position 562, 585, 881, 920, 1087, 1813, 1947, 2295, 2652, 3046, 3484 oder/und 3594 der in SEQ ID No. 1 gezeigten Nukleotidsequenz oder
(b) an Position 410, 484, 598, 1012, 1435, 1583, 1808 oder/und 2301 der in SEQ ID No. 5 gezeigten Nukleotidsequenz lokalisiert ist.

5. Verfahren nach einem der Ansprüche 3 bis 4,
**dadurch gekennzeichnet,**
**daß** die Insertionen ausgewählt werden aus Nukleotidsequenzen, die für Cysteinreste, Bereiche mit mehreren geladenen Aminosäuren oder Tyr-Resten, DNA-bindende Epitope, metallbindende Epitope, immunogene Epitope, allergene Epitope, antigene Epitope, Streptavidin, Enzyme, Cytokine oder Antikörper-bindende Proteine kodieren.

6. Verfahren nach einem der Ansprüche 1 bis 5,
**dadurch gekennzeichnet,**
**daß** die für das Signalpeptid kodierende Nukleinsäure
(a) den Signalpeptid-kodierenden Abschnitt der in SEQ ID No. 7 dargestellten Nukleotidsequenz, oder/und
(b) eine der Sequenz aus (a) im Rahmen der Degeneration des genetischen Codes entsprechende Nukleotidsequenz
umfaßt.

7. Verfahren nach einem der Ansprüche 1 bis 6,
**dadurch gekennzeichnet,**
**daß** in der Wirtszelle mindestens zwei S-Layer-Gene exprimiert werden, wobei eines davon für ein modifiziertes S-Layer-Protein und ein anderes für ein nichtmodifiziertes S-Layer-Protein kodiert.

8. Verfahren nach Anspruch 7,
**dadurch gekennzeichnet,**
**daß** das modifizierte S-Layer-Protein in der Lage ist, eine mit dem nichtmodifiziertenS-Layer-ProteinkompatibleS-Layer-Strukturauszubilden.

9. Nukleinsäure,
**dadurch gekennzeichnet,**
**daß** sie für ein gegebenenfalls heterologe Peptid- oder Polypeptidinsertionen enthaltendes S-Layer-Protein kodiert ausgewählt aus SbsA oder SbsB und in operativer Verknüpfung mit einer MalE-Signalsequenz ist, die für ein Peptid kodiert, das eine Sekretion des S-Layer-Proteins in den periplasmatischen Raum einer gram-negativen prokaryontischen Wirtszelle bewirkt,
und die Signalsequenz 5'-seitig von der S-Layer-Protein-kodierenden Nukleinsäure angeordnet ist.

10. Vektor,
**dadurch gekennzeichnet,**
**daß** er mindestens eine Kopie einer Nukleinsäure nach Anspruch 9 enthält.

11. Gram-negative prokaryontische Zelle,
**dadurch gekennzeichnet,**
**daß** sie mit einer Nukleinsäure nach Anspruch 9 oder einem Vektor nach Anspruch 10 transformiert ist.

12. Prokaryontische Zelle nach Anspruch 11,
**dadurch gekennzeichnet,**
**daß** sie eine S-Layer-Struktur im periplasmatischen Raum enthält.

## Claims

1. Process for the heterologous production of the S-layer proteins SbsA or SbsB, **characterized in that**
(a) a Gram-negative prokaryotic host cell which is transformed with a nucleic acid which codes for an S-layer protein and is operatively linked on the 5' side of the nucleic acid to the MalE signal sequence which codes for a peptide which brings about secretion of the S-layer protein into the periplasmic space of the host cell is prepared,
(b) the host cell is cultivated under conditions leading to expression of the nucleic acid and to production of the polypeptide encoded thereby, and
(c) where appropriate the resulting polypeptide is isolated from the periplasmic space of the host cell.

2. Process according to Claim 1, **characterized in that** the nucleic acid which codes for an S-layer protein is selected from
(i) a nucleic acid which comprises the nucleotide sequence shown in SEQ ID NO.1 from position 91 to 3684,
(ii) a nucleic acid which comprises the nucleotide sequence shown in SEQ ID NO. 5 from position 94 to 2763,
(iii) a nucleic acid which comprises a nucleotide sequence corresponding to the nucleic acid from (i) or (ii) within the scope of the degeneracy of the genetic code, and
(iv) a nucleic acid which comprises a nucleotide sequence hybridizing with the nucleic acids from (i), (ii) or/and (iii) under stringent conditions.

3. Process according to either of Claims 1 and 2, **characterized in that** the nucleic acid coding for the S-layer protein comprises one or more insertions which code for heterologous peptide or polypeptide sequences.

4. Process according to Claim 3, **characterized in that** the insertion site is located
(a) at position 562, 585, 881, 920, 1087, 1813, 1947, 2295, 2652, 3046, 3484 or/and 3594 of the nucleotide sequence shown in SEQ ID NO. 1 or
(b) at position 410, 484, 598, 1012, 1435, 1583, 1808 or/and 2301 of the nucleotide sequence shown in SEQ ID NO. 5.

5. Process according to either of Claims 3 and 4, **characterized in that** the insertions are selected from nucleotide sequences which code for cysteine residues, regions with a plurality of charged amino acids or Tyr residues, DNA-binding epitopes, metal-binding epitopes, immunogenic epitopes, allergenic epitopes, antigenic epitopes, streptavidin, enzymes, cytokines or antibody-binding proteins.

6. Process according to any of Claims 1 to 5, **characterized in that** the nucleic acid coding for the signal peptide comprises
(a) the signal peptide-encoding section of the nucleotide sequence depicted in SEQ ID NO.7 or/and,
(b) a nucleotide sequence corresponding to the sequence from (a) within the scope of the degeneracy of the genetic code.

7. Process according to any of Claims 1 to 6, **characterized in that** at least two S-layer genes are expressed in the host cell, one of which codes for a modified S-layer protein and another codes for an unmodified S-layer protein.

8. Process according to Claim 7, **characterized in that** the modified S-layer protein is able to form an S-layer structure which is compatible with the unmodified S-layer protein.

9. Nucleic acid, **characterized in that** it codes for an S-layer protein optionally comprising heterologous peptide or polypeptide insertions selected from SbsA or SbsB and is operatively linked to a MalE signal sequence which codes for a peptide which brings about secretion of the S-layer protein into the periplasmic space of a Gram-negative prokaryotic host cell, and the signal sequence is disposed on the 5' side of the S-layer protein-encoding nucleic acid.

10. Vector, **characterized in that** it comprises at least one copy of a nucleic acid according to Claim 9.

11. Gram-negative prokaryotic cell, **characterized in that** it is transformed with a nucleic acid according to Claim 9 or a vector according to Claim 10.

12. Prokaryotic cell according to Claim 11, **characterized in that** it comprises an S-layer structure in the periplasmic space.

## Revendications

1. Procédé de préparation hétérologue des protéines de couche S SbsA ou SbsB, **caractérisé en ce qu'**
(a) on fournit une cellule hôte procaryote Gram négative laquelle est transformée avec un acide nucléique codant la protéine de couche S, dans une association opérationnelle côté 5' de l'acide nucléique, avec la séquence signal MalE codant un peptide qui provoque une sécrétion de la protéine de couche S dans l'espace périplasmique de la cellule hôte,
(b) on cultive la cellule hôte dans des conditions qui conduisent à une expression de l'acide nucléique et à une production du polypeptide codé par celui-ci et
(c) on obtient, le cas échéant, le polypeptide qui en résulte à partir d'un espace périplasmique de la cellule hôte.

2. Procédé selon la revendication 1, **caractérisé en ce qu'**on choisit l'acide nucléique, qui code une protéine de couche S, parmi
(i) un acide nucléique qui comprend la séquence de nucléotides représentée dans la SEQ ID No. 1 de la position 91 à 3684,
(ii) un acide nucléique qui comprend la séquence de nucléotides représentée dans la SEQ ID No. 5 de la position 94 à 2763,
(iii) un acide nucléique qui comprend une séquence de nucléotides correspondant à l'acide nucléique de (i) ou de (ii) dans le cadre de la dégénérescence du code génétique et
(iv) un acide nucléique qui comprend une séquence de nucléotides s'hybridant avec les acides nucléiques de (i), (ii) et/ou (iii) dans des conditions stringentes.

3. Procédé selon l'une quelconque des revendications 1 à 2, **caractérisé en ce que** l'acide nucléique codant la protéine de couche S contient une ou plusieurs insertions qui codent des séquences hétérologues de peptides ou de polypeptides.

4. Procédé selon la revendication 3, **caractérisé en ce que** la zone d'insertion est localisée
(a) sur les positions 562, 585, 881, 920, 1087, 1813, 1947, 2295, 2652, 3046, 3484 et/ou 3594 de la séquence de nucléotides représentée dans SEQ ID No. 1 ou
(b) sur les positions 410, 484, 598, 1012, 1435, 1583, 1808 et/ou 2301 de la séquence de nucléotides représentée dans SEQ ID No. 5.

5. Procédé selon l'une quelconque des revendications 3 à 4, **caractérisé en ce que** les insertions sont choisies parmi les séquences de nucléotides qui codent les radicaux cystéine, des zones comportant plusieurs acides aminés chargés ou des radicaux tyrosine, des épitopes liant l'ADN, des épitopes liant les métaux, des épitopes immunogènes, des épitopes allergènes, des épitopes antigènes, la streptavidine, des enzymes, des cytokines ou des protéines liant les anticorps.

6. Procédé selon l'une quelconque des revendications 1 à 5, **caractérisé en ce que** l'acide nucléique codant le peptide signal comprend
(a) le segment codant le peptide signal de la séquence de nucléotides représentée dans SEQ ID No. 7 et/ou
(b) une séquence de nucléotides correspondant à la séquence de (a) dans le cadre de la dégénérescence du code génétique.

7. Procédé selon l'une quelconque des revendications 1 à 6, **caractérisé en ce qu'**au moins deux gènes de couche S sont exprimés dans la cellule hôte, l'un d'entre eux codant une protéine de couche S modifiée et l'autre une protéine de couche S non modifiée.

8. Procédé selon la revendication 7, **caractérisé en ce que** la protéine de couche S modifiée est en mesure de constituer, avec la protéine de couche S non modifiée, une structure de couche S compatible.

9. Acide nucléique **caractérisé en ce qu'**il code une protéine de couche S contenant le cas échéant des insertions de peptides ou de polypeptides hétérologues, choisie parmi SbsA ou SbsB et se trouve en association opérationnelle avec une séquence signal MaLE, qui code un peptide qui provoque une sécrétion de la protéine de couche S dans l'espace périplasmique d'une cellule hôte procaryote Gram négative, et la séquence signal est disposée côté 5' de l'acide nucléique codant la protéine de couche S.

10. Vecteur **caractérisé en ce qu'**il contient au moins une copie d'un acide nucléique selon la revendication 9.

11. Cellule procaryote Gram négative, **caractérisée en ce qu'**elle est transformée avec un acide nucléique selon la revendication 9 ou un vecteur selon la revendication 10.

12. Cellule procaryote selon la revendication 11, **caractérisée en ce qu'**elle contient une structure de couche S dans l'espace périplasmique.


### SEQUENZPROTOKOLL

(1) ALLGEMEINE ANGABEN:
   (i) ANMELDER:
      (A) NAME: Werner Lubitz
      (B) STRASSE: Schoenborngasse 12/7
      (C) ORT: Wien
      (E) LAND: Austria
      (F) POSTLEITZAHL: 1080
   (ii) BEZEICHNUNG DER ERFINDUNG: Sekretion von S-Layer-Proteinen in das Periplasma und in den extrazellulären Raum
   (iii) ANZAHL DER SEQUENZEN: 6
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.30 (EPA)
(2) ANGABEN ZU SEQ ID NO: 1:
   (i) SEQUENZKENNZEICHEN:
      (A) LÄNGE: 3687 Basenpaare
      (B) ART: Nucleotid
      (C) STRANGFORM: beides
      (D) TOPOLOGIE: linear
   (vi) URSPRÜNGLICHE HERKUNFT:
      (A) ORGANISMUS: Bacillus stearothermophilus
      (B) STAMM: PV72
   (vii) UNMITTELBARE HERKUNFT:
      (B) CLON(E): sbsA
   (ix) MERKMAL:
      (A) NAME/SCHLÜSSEL: CDS
      (B) LAGE:1..3684
   (ix) MERKMAL:
      (A) NAME/SCHLÜSSEL: sig_peptide
      (B) LAGE:1..90
   (ix) MERKMAL:
      (A) NAME/SCHLÜSSEL: mat_peptide
      (B) LAGE:91..3684
   (xi) SEQUENZBESCHREIBUNG: SEQ ID NO: 1:
   ATG GAT AGG AAA AAA GCT GTG AAA CTA GCA ACA GCA AGT GCT ATT GCA      48
   Met Asp Arg Lys Lys Ala Val Lys Leu Ala Thr Ala Ser Ala Ile Ala
   -30                 -25                 -20                 -15
   GCA AGT GCA TTT GTC GCT GCA AAT CCA AAC GCT TCT GAA GCG GCT ACA      96
   Ala Ser Ala Phe Val Ala Ala Asn Pro Asn Ala Ser Glu Ala Ala Thr
                   -10                 -5                  1
   GAT GTA GCA ACA GTA GTA AGC CAA GCA AAA GCA CAG TTC AAA AAA GCA     144
   Asp Val Ala Thr Val Val Ser Gln Ala Lys Ala Gln Phe Lys Lys Ala
               5                   10                  15
   TAC TAT ACT TAC AGC CAT ACA GTA ACG GAA ACT GGT GAA TTC CCA AAC     192
   Tyr Tyr Thr Tyr Ser His Thr Val Thr Glu Thr Gly Glu Phe Pro Asn
       20                  25                  30
   ATT AAC GAT GTA TAT GCT GAA TAC AAC AAA GCG AAA AAA CGA TAC CGT     240
   Ile Asn Asp Val Tyr Ala Glu Tyr Asn Lys Ala Lys Lys Arg Tyr Arg
   35                  40                  45                  50
   GAT GCG GTA GCA TTA GTG AAT AAA GCA GGT GGC GCG AAA AAA GAC GCT     288
   Asp Ala Val Ala Leu Val Asn Lys Ala Gly Gly Ala Lys Lys Asp Ala
                   55                  60                  65
   TAC TTA GCT GAT TTA CAA AAA GAA TAT GAA ACT TAC GTT TTC AAA GCA     336
   Tyr Leu Ala Asp Leu Gln Lys Glu Tyr Glu Thr Tyr Val Phe Lys Ala
               70                  75                  80
   AAC CCT AAA TCT GGC GAA GCT CGT GTA GCA ACT TAC ATC GAT GCT TAC     384
   Asn Pro Lys Ser Gly Glu Ala Arg Val Ala Thr Tyr Ile Asp Ala Tyr
           85                  90                  95
   AAC TAT GCA ACA AAA TTA GAC GAA ATG CGC CAA GAG CTA GAG GCT GCT     432
   Asn Tyr Ala Thr Lys Leu Asp Glu Met Arg Gln Glu Leu Glu Ala Ala
       100                 105                 110
   GTT CAA GCA AAA GAT TTA GAA AAA GCA GAA CAA TAC TAT CAC AAA ATT     480
   Val Gln Ala Lys Asp Leu Glu Lys Ala Glu Gln Tyr Tyr His Lys Ile
   115                 120                 125                 130
   CCT TAT GAA ATT AAA ACT CGC ACA GTC ATT TTA GAT CGC GTA TAT GGT     528
   Pro Tyr Glu Ile Lys Thr Arg Thr Val Ile Leu Asp Arg Val Tyr Gly
                   135                 140                 145
   AAA ACA ACT CGT GAT TTA CTT CGC TCT ACA TTT AAA GCA AAA GCA CAA     576
   Lys Thr Thr Arg Asp Leu Leu Arg Ser Thr Phe Lys Ala Lys Ala Gln
               150                 155                 160
   GAA CTT CGC GAC AGC TTA ATT TAT GAT ATT ACC GTT GCA ATG AAA GCG     624
   Glu Leu Arg Asp Ser Leu Ile Tyr Asp Ile Thr Val Ala Met Lys Ala
           165                 170                 175
   CGC GAA GTA CAA GAC GCT GTG AAA GCA GGC AAT TTA GAC AAA GCT AAA     672
   Arg Glu Val Gln Asp Ala Val Lys Ala Gly Asn Leu Asp Lys Ala Lys
       180                 185                 190
   GCT GCT GTT GAT CAA ATC AAT CAA TAC TTA CCA AAA GTA ACA GAT GCT     720
   Ala Ala Val Asp Gln Ile Asn Gln Tyr Leu Pro Lys Val Thr Asp Ala
   195                 200                 205                 210
   TTC AAA ACT GAA CTA ACA GAA GTA GCG AAA AAA GCA TTA GAT GCA GAT     768
   Phe Lys Thr Glu Leu Thr Glu Val Ala Lys Lys Ala Leu Asp Ala Asp
                   215                 220                 225
   GAA GCT GCG CTT ACT CCA AAA GTT GAA AGT GTA AGT GCG ATT AAC ACT      816
   Glu Ala Ala Leu Thr Pro Lys Val Glu Ser Val Ser Ala Ile Asn Thr
               230                 235                 240
   CAA AAC AAA GCT GTT GAA TTA ACA GCA GTA CCA GTG AAC GGA ACA CTA      864
   Gln Asn Lys Ala Val Glu Leu Thr Ala Val Pro Val Asn Gly Thr Leu
           245                 250                 255
   AAA TTA CAA CTT TCA GCT GCT GCA AAT GAA GAT ACA GTA AAC GTA AAT      912
   Lys Leu Gln Leu Ser Ala Ala Ala Asn Glu Asp Thr Val Asn Val Asn
       260                 265                 270
   ACT GTA CGT ATC TAT AAA GTG GAC GGT AAC ATT CCA TTT GCC CTT AAT      960
   Thr Val Arg Ile Tyr Lys Val Asp Gly Asn Ile Pro Phe Ala Leu Asn
   275                 280                 285                 290
   ACG GCA GAT GTT TCT TTA TCT ACA GAC GGA AAA ACT ATC ACT GTG GAT     1008
   Thr Ala Asp Val Ser Leu Ser Thr Asp Gly Lys Thr Ile Thr Val Asp
                   295                 300                 305
   GCT TCA ACT CCA TTC GAA AAT AAT ACG GAG TAT AAA GTA GTA GTT AAA     1056
   Ala Ser Thr Pro Phe Glu Asn Asn Thr Glu Tyr Lys Val Val Val Lys
               310                 315                 320
   GGT ATT AAA GAC AAA AAT GGC AAA GAA TTT AAA GAA GAT GCA TTC ACT     1104
   Gly Ile Lys Asp Lys Asn Gly Lys Glu Phe Lys Glu Asp Ala Phe Thr
           325                 330                 335
   TTC AAG CTT CGA AAT GAT GCT GTA GTT ACT CAA GTG TTT GGA ACT AAT     1152
   Phe Lys Leu Arg Asn Asp Ala Val Val Thr Gln Val Phe Gly Thr Asn
       340                 345                 350
   GTA ACA AAC AAC ACT TCT GTA AAC TTA GCA GCA GGT ACT TTC GAC ACT     1200
   Val Thr Asn Asn Thr Ser Val Asn Leu Ala Ala Gly Thr Phe Asp Thr
   355                 360                 365                 370
   GAC GAT ACT TTA ACA GTA GTA TTT GAT AAG TTG TTA GCA CCT GAA ACT     1248
   Asp Asp Thr Leu Thr Val Val Phe Asp Lys Leu Leu Ala Pro Glu Thr
                   375                 380                 385
   GTA AAC AGC TCG AAC GTT ACT ATT ACA GAT GTT GAA ACT GGA AAA CGC     1296
   Val Asn Ser Ser Asn Val Thr Ile Thr Asp Val Glu Thr Gly Lys Arg
               390                 395                 400
   ATT CCA GTA ATT GCA TCT ACT TCT GGT TCT ACA ATT ACT ATT ACG TTA     1344
   Ile Pro Val Ile Ala Ser Thr Ser Gly Ser Thr Ile Thr Ile Thr Leu
           405                 410                 415
   AAA GAA GCG TTA GTA ACT GGT AAA CAA TAT AAA CTT GCT ATC AAT AAT     1392
   Lys Glu Ala Leu Val Thr Gly Lys Gln Tyr Lys Leu Ala Ile Asn Asn
       420                 425                 430
   GTT AAA ACA TTA ACT GGT TAC AAT GCA GAA GCT TAC GAG TTA GTG TTC     1440
   Val Lys Thr Leu Thr Gly Tyr Asn Ala Glu Ala Tyr Glu Leu Val Phe
   435                 440                 445                 450
   ACT GCA AAC GCA TCA GCA CCA ACT GTT GCT ACC GCT CCT ACT ACT TTA     1488
   Thr Ala Asn Ala Ser Ala Pro Thr Val Ala Thr Ala Pro Thr Thr Leu
                   455                 460                 465
   GGT GGT ACA ACT TTA TCT ACT GGT TCT CTT ACA ACA AAT GTT TGG GGT     1536
   Gly Gly Thr Thr Leu Ser Thr Gly Ser Leu Thr Thr Asn Val Trp Gly
               470                 475                 480
   AAA TTG GCT GGT GGT GTG AAT GAA GCT GGA ACT TAT TAT CCT GGT CTT     1584
   Lys Leu Ala Gly Gly Val Asn Glu Ala Gly Thr Tyr Tyr Pro Gly Leu
           485                 490                 495
   CAA TTC ACA ACA ACG TTT GCT ACT AAG TTA GAC GAA TCT ACT TTA GCT     1632
   Gln Phe Thr Thr Thr Phe Ala Thr Lys Leu Asp Glu Ser Thr Leu Ala
       500                 505                 510
   GAT AAC TTT GTA TTA GTT GAA AAA GAA TCT GGT ACA GTT GTT GCT TCT     1680
   Asp Asn Phe Val Leu Val Glu Lys Glu Ser Gly Thr Val Val Ala Ser
   515                 520                 525                 530
   GAA CTA AAA TAT AAT GCA GAC GCT AAA ATG GTA ACT TTA GTG CCA AAA     1728
   Glu Leu Lys Tyr Asn Ala Asp Ala Lys Met Val Thr Leu Val Pro Lys
                   535                 540                 545
   GCG GAC CTT AAA GAA AAT ACA ATC TAT CAA ATC AAA ATT AAA AAA GGC     1776
   Ala Asp Leu Lys Glu Asn Thr Ile Tyr Gln Ile Lys Ile Lys Lys Gly
               550                 555                 560
   TTG AAG TCC GAT AAA GGT ATT GAA TTA GGC ACT GTT AAC GAG AAA ACA     1824
   Leu Lys Ser Asp Lys Gly Ile Glu Leu Gly Thr Val Asn Glu Lys Thr
           565                 570                 575
   TAT GAG TTC AAA ACT CAA GAC TTA ACT GCT CCT ACA GTT ATT AGC GTA     1872
   Tyr Glu Phe Lys Thr Gln Asp Leu Thr Ala Pro Thr Val Ile Ser Val
       580                 585                 590
   ACG TCT AAA AAT GGC GAC GCT GGA TTA AAA GTA ACT GAA GCT CAA GAA     1920
   Thr Ser Lys Asn Gly Asp Ala Gly Leu Lys Val Thr Glu Ala Gln Glu
   595                 600                 605                 610
   TTT ACT GTG AAG TTC TCA GAG AAT TTA AAT ACA TTT AAT GCT ACA ACC     1968
   Phe Thr Val Lys Phe Ser Glu Asn Leu Asn Thr Phe Asn Ala Thr Thr
                   615                 620                 625
   GTT TCG GGT AGC ACA ATC ACA TAC GGT CAA GTT GCT GTA GTA AAA GCG     2016
   Val Ser Gly Ser Thr Ile Thr Tyr Gly Gln Val Ala Val Val Lys Ala
               630                 635                 640
   GGT GCA AAC TTA TCT GCT CTT ACA GCA AGT GAC ATC ATT CCA GCT AGT     2064
   Gly Ala Asn Leu Ser Ala Leu Thr Ala Ser Asp Ile Ile Pro Ala Ser
           645                 650                 655
   GTT GAA GCG GTT ACT GGT CAA GAT GGA ACA TAC AAA GTG AAA GTT GCT     2112
   Val Glu Ala Val Thr Gly Gln Asp Gly Thr Tyr Lys Val Lys Val Ala
       660                 665                 670
   GCT AAC CAA TTA GAA CGT AAC CAA GGG TAC AAA TTA GTA GTG TTC GGT     2160
   Ala Asn Gln Leu Glu Arg Asn Gln Gly Tyr Lys Leu Val Val Phe Gly
   675                 680                 685                 690
   AAA GGT GCA ACA GCT CCT GTT AAA GAT GCT GCA AAT GCA AAT ACT TTA     2208
   Lys Gly Ala Thr Ala Pro Val Lys Asp Ala Ala Asn Ala Asn Thr Leu
                   695                 700                 705
   GCA ACT AAC TAT ATC TAT ACA TTT ACA ACT GAA GGT CAA GAC GTA ACA     2256
   Ala Thr Asn Tyr Ile Tyr Thr Phe Thr Thr Glu Gly Gln Asp Val Thr
               710                 715                 720
   GCA CCA ACG GTT ACA AAA GTA TTC AAA GGT GAT TCT TTA AAA GAC GCT     2304
   Ala Pro Thr Val Thr Lys Val Phe Lys Gly Asp Ser Leu Lys Asp Ala
           725                 730                 735
   GAT GCA GTT ACT ACA CTT ACG AAC GTT GAT GCA GGT CAA AAA TTC ACT     2352
   Asp Ala Val Thr Thr Leu Thr Asn Val Asp Ala Gly Gln Lys Phe Thr
       740                 745                 750
   ATC CAA TTT AGC GAA GAA TTA AAA ACT TCT AGT GGT TCT TTA GTG GGT     2400
   Ile Gln Phe Ser Glu Glu Leu Lys Thr Ser Ser Gly Ser Leu Val Gly
   755                 760                 765                 770
   GGC AAA GTA ACT GTC GAG AAA TTA ACA AAC AAC GGA TGG GTA GAT GCT     2448
   Gly Lys Val Thr Val Glu Lys Leu Thr Asn Asn Gly Trp Val Asp Ala
                   775                 780                 785
   GGT ACT GGA ACA ACT GTA TCA GTT GCT CCT AAG ACA GAT GCA AAT GGT     2496
   Gly Thr Gly Thr Thr Val Ser Val Ala Pro Lys Thr Asp Ala Asn Gly
               790                 795                 800
   AAA GTA ACA GCT GCT GTG GTT ACA TTA ACT GGT CTT GAC AAT AAC GAC     2544
   Lys Val Thr Ala Ala Val Val Thr Leu Thr Gly Leu Asp Asn Asn Asp
           805                 810                 815
   AAA GAT GCG AAA TTG CGT CTG GTA GTA GAT AAG TCT TCT ACT GAT GGA     2592
   Lys Asp Ala Lys Leu Arg Leu Val Val Asp Lys Ser Ser Thr Asp Gly
       820                 825                 830
   ATT GCT GAT GTA GCT GGT AAT GTA ATT AAG GAA AAA GAT ATT TTA ATT     2640
   Ile Ala Asp Val Ala Gly Asn Val Ile Lys Glu Lys Asp Ile Leu Ile
   835                 840                 845                 850
   CGT TAC AAC AGC TGG AGA CAC ACT GTA GCT TCT GTG AAA GCT GCT GCT     2688
   Arg Tyr Asn Ser Trp Arg His Thr Val Ala Ser Val Lys Ala Ala Ala
                   855                 860                 865
   GAC AAA GAT GGT CAA AAC GCT TCT GCT GCA TTC CCA ACA AGC ACT GCA     2736
   Asp Lys Asp Gly Gln Asn Ala Ser Ala Ala Phe Pro Thr Ser Thr Ala
               870                 875                 880
   ATT GAT ACA ACT AAG AGC TTA TTA GTT GAA TTC AAT GAA ACT GAT TTA     2784
   Ile Asp Thr Thr Lys Ser Leu Leu Val Glu Phe Asn Glu Thr Asp Leu
           885                 890                 895
   GCG GAA GTT AAA CCT GAG AAC ATC GTT GTT AAA GAT GCA GCA GGT AAT     2832
   Ala Glu Val Lys Pro Glu Asn Ile Val Val Lys Asp Ala Ala Gly Asn
       900                 905                 910
   GCG GTA GCT GGT ACT GTA ACA GCA TTA GAC GGT TCT ACA AAT AAA TTT     2880
   Ala Val Ala Gly Thr Val Thr Ala Leu Asp Gly Ser Thr Asn Lys Phe
   915                 920                 925                 930
   GTA TTC ACT CCA TCT CAA GAA TTA AAA GCT GGT ACA GTT TAC TCT GTA     2928
   Val Phe Thr Pro Ser Gln Glu Leu Lys Ala Gly Thr Val Tyr Ser Val
                   935                 940                 945
   ACA ATT GAC GGT GTG AGA GAT AAA GTA GGT AAC ACA ATC TCT AAA TAC     2976
   Thr Ile Asp Gly Val Arg Asp Lys Val Gly Asn Thr Ile Ser Lys Tyr
               950                 955                 960
   ATT ACT TCG TTC AAG ACT GTA TCT GCG AAT CCA ACG TTA TCT TCA ATC     3024
   Ile Thr Ser Phe Lys Thr Val Ser Ala Asn Pro Thr Leu Ser Ser Ile
           965                 970                 975
   AGC ATT GCT GAC GGT GCA GTT AAC GTT GAC CGT TCT AAA ACA ATT ACA     3072
   Ser Ile Ala Asp Gly Ala Val Asn Val Asp Arg Ser Lys Thr Ile Thr
       980                 985                 990
   ATT GAA TTC AGC GAT TCA GTT CCA AAC CCA ACA ATC ACT CTT AAG AAG     3120
   Ile Glu Phe Ser Asp Ser Val Pro Asn Pro Thr Ile Thr Leu Lys Lys
   995                 1000                1005                1010
   GCT GAC GGA ACT TCA TTT ACT AAT TAC ACT TTA GTA AAT GTA AAT AAT     3168
   Ala Asp Gly Thr Ser Phe Thr Asn Tyr Thr Leu Val Asn Val Asn Asn
                   1015                1020                1025
   GAA AAT AAA ACA TAC AAA ATT GTA TTC CAC AAA GGT GTA ACA CTT GAC     3216
   Glu Asn Lys Thr Tyr Lys Ile Val Phe His Lys Gly Val Thr Leu Asp
               1030                1035                1040
   GAG TTT ACT CAA TAT GAG TTA GCA GTT TCA AAA GAT TTT CAA ACT GGT     3264
   Glu Phe Thr Gln Tyr Glu Leu Ala Val Ser Lys Asp Phe Gln Thr Gly
           1045                1050                1055
   ACT GAT ATT GAT AGC AAA GTT ACA TTC ATC ACA GGT TCT GTT GCT ACT     3312
   Thr Asp Ile Asp Ser Lys Val Thr Phe Ile Thr Gly Ser Val Ala Thr
       1060                1065                1070
   GAC GAA GTA AAA CCT GCT CTA GTA GGC GTT GGT TCA TGG AAT GGA ACA     3360
   Asp Glu Val Lys Pro Ala Leu Val Gly Val Gly Ser Trp Asn Gly Thr
   1075                1080                1085                1090
   AGC TAT ACT CAG GAT GCT GCA GCA ACA CGA CTT CGG TCT GTA GCT GAC      3408
   Ser Tyr Thr Gln Asp Ala Ala Ala Thr Arg Leu Arg Ser Val Ala Asp
                   1095                1100                1105
   TTC GTT GCG GAG CCA GTT GCC CTT CAA TTC TCA GAA GGT ATC GAT TTA      3456
   Phe Val Ala Glu Pro Val Ala Leu Gln Phe Ser Glu Gly Ile Asp Leu
               1110                1115                1120
   ACG AAT GCA ACT GTG ACA GTA ACA AAT ATT ACT GAT GAT AAA ACT GTT      3504
   Thr Asn Ala Thr Val Thr Val Thr Asn Ile Thr Asp Asp Lys Thr Val
           1125                1130                1135
   GAA GTT ATT TCA AAA GAG AGT GTA GAC GCA GAC CAT GAT GCA GGT GCT      3552
   Glu Val Ile Ser Lys Glu Ser Val Asp Ala Asp His Asp Ala Gly Ala
       1140                1145                1150
   ACT AAG GAG ACA TTA GTA ATT AAC ACA GTT ACT CCT TTA GTA CTT GAT      3600
   Thr Lys Glu Thr Leu Val Ile Asn Thr Val Thr Pro Leu Val Leu Asp
   1155                1160                1165                1170
   AAC AGC AAG ACT TAT AAG ATT GTT GTA AGT GGA GTT AAA GAT GCA GCA      3648
   Asn Ser Lys Thr Tyr Lys Ile Val Val Ser Gly Val Lys Asp Ala Ala
                   1175                1180                1185
   GGT AAT GTT GCA GAT ACT ATT ACA TTC TAT ATT AAG TAA                  3687
   Gly Asn Val Ala Asp Thr Ile Thr Phe Tyr Ile Lys
               1190                1195
(2) ANGABEN ZU SEQ ID NO: 2:
   (i) SEQUENZKENNZEICHEN:
      (A) LÄNGE: 1228 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Asp Arg Lys Lys Ala Val Lys Leu Ala Thr Ala Ser Ala Ile Ala
   -30                 -25                 -20                 -15
   Ala Ser Ala Phe Val Ala Ala Asn Pro Asn Ala Ser Glu Ala Ala Thr
                   -10                 -5                   1
   Asp Val Ala Thr Val Val Ser Gln Ala Lys Ala Gln Phe Lys Lys Ala
             5                  10                  15
   Tyr Tyr Thr Tyr Ser His Thr Val Thr Glu Thr Gly Glu Phe Pro Asn
        20                  25                  30
   Ile Asn Asp Val Tyr Ala Glu Tyr Asn Lys Ala Lys Lys Arg Tyr Arg
    35                  40                  45                  50
   Asp Ala Val Ala Leu Val Asn Lys Ala Gly Gly Ala Lys Lys Asp Ala
                    55                  60                  65
   Tyr Leu Ala Asp Leu Gln Lys Glu Tyr Glu Thr Tyr Val Phe Lys Ala
                70                  75                  80
   Asn Pro Lys Ser Gly Glu Ala Arg Val Ala Thr Tyr Ile Asp Ala Tyr
            85                  90                  95
   Asn Tyr Ala Thr Lys Leu Asp Glu Met Arg Gln Glu Leu Glu Ala Ala
       100                 105                 110
   Val Gln Ala Lys Asp Leu Glu Lys Ala Glu Gln Tyr Tyr His Lys Ile
   115 120 125 130
   Pro Tyr Glu Ile Lys Thr Arg Thr Val Ile Leu Asp Arg Val Tyr Gly
   135 140 145
   Lys Thr Thr Arg Asp Leu Leu Arg Ser Thr Phe Lys Ala Lys Ala Gln
   150 155 160
   Glu Leu Arg Asp Ser Leu Ile Tyr Asp Ile Thr Val Ala Met Lys Ala
   165 170 175
   Arg Glu Val Gln Asp Ala Val Lys Ala Gly Asn Leu Asp Lys Ala Lys
   180 185 190
   Ala Ala Val Asp Gln Ile Asn Gln Tyr Leu Pro Lys Val Thr Asp Ala
   195 200 205 210
   Phe Lys Thr Glu Leu Thr Glu Val Ala Lys Lys Ala Leu Asp Ala Asp
   215 220 225
   Glu Ala Ala Leu Thr Pro Lys Val Glu Ser Val Ser Ala Ile Asn Thr
   230 235 240
   Gln Asn Lys Ala Val Glu Leu Thr Ala Val Pro Val Asn Gly Thr Leu
   245 250 255
   Lys Leu Gln Leu Ser Ala Ala Ala Asn Glu Asp Thr Val Asn Val Asn
   260 265 270
   Thr Val Arg Ile Tyr Lys Val Asp Gly Asn Ile Pro Phe Ala Leu Asn
   275 280 285 290
   Thr Ala Asp Val Ser Leu Ser Thr Asp Gly Lys Thr Ile Thr Val Asp
   295 300 305
   Ala Ser Thr Pro Phe Glu Asn Asn Thr Glu Tyr Lys Val Val Val Lys
   310 315 320
   Gly Ile Lys Asp Lys Asn Gly Lys Glu Phe Lys Glu Asp Ala Phe Thr
   325 330 335
   Phe Lys Leu Arg Asn Asp Ala Val Val Thr Gln Val Phe Gly Thr Asn
   340 345 350
   Val Thr Asn Asn Thr Ser Val Asn Leu Ala Ala Gly Thr Phe Asp Thr
   355 360 365 370
   Asp Asp Thr Leu Thr Val Val Phe Asp Lys Leu Leu Ala Pro Glu Thr
   375 380 385
   Val Asn Ser Ser Asn Val Thr Ile Thr Asp Val Glu Thr Gly Lys Arg
   390 395 400
   Ile Pro Val Ile Ala Ser Thr Ser Gly Ser Thr Ile Thr Ile Thr Leu
   405 410 415
   Lys Glu Ala Leu Val Thr Gly Lys Gln Tyr Lys Leu Ala Ile Asn Asn
   420 425 430
   Val Lys Thr Leu Thr Gly Tyr Asn Ala Glu Ala Tyr Glu Leu Val Phe
   435 440 445 450
   Thr Ala Asn Ala Ser Ala Pro Thr Val Ala Thr Ala Pro Thr Thr Leu
   455 460 465
   Gly Gly Thr Thr Leu Ser Thr Gly Ser Leu Thr Thr Asn Val Trp Gly
               470                 475                 480
   Lys Leu Ala Gly Gly Val Asn Glu Ala Gly Thr Tyr Tyr Pro Gly Leu
           485                 490                 495
   Gln Phe Thr Thr Thr Phe Ala Thr Lys Leu Asp Glu Ser Thr Leu Ala
       500                 505                 510
   Asp Asn Phe Val Leu Val Glu Lys Glu Ser Gly Thr Val Val Ala Ser
   515                 520                 525                 530
   Glu Leu Lys Tyr Asn Ala Asp Ala Lys Met Val Thr Leu Val Pro Lys
                   535                 540                 545
   Ala Asp Leu Lys Glu Asn Thr Ile Tyr Gln Ile Lys Ile Lys Lys Gly
               550                 555                 560
   Leu Lys Ser Asp Lys Gly Ile Glu Leu Gly Thr Val Asn Glu Lys Thr
           565                 570                 575
   Tyr Glu Phe Lys Thr Gln Asp Leu Thr Ala Pro Thr Val Ile Ser Val
       580                 585                 590
   Thr Ser Lys Asn Gly Asp Ala Gly Leu Lys Val Thr Glu Ala Gln Glu
   595                 600                 605                 610
   Phe Thr Val Lys Phe Ser Glu Asn Leu Asn Thr Phe Asn Ala Thr Thr
                   615                 620                 625
   Val Ser Gly Ser Thr Ile Thr Tyr Gly Gln Val Ala Val Val Lys Ala
               630                 635                 640
   Gly Ala Asn Leu Ser Ala Leu Thr Ala Ser Asp Ile Ile Pro Ala Ser
           645                 650                 655
   Val Glu Ala Val Thr Gly Gln Asp Gly Thr Tyr Lys Val Lys Val Ala
       660                 665                 670
   Ala Asn Gln Leu Glu Arg Asn Gln Gly Tyr Lys Leu Val Val Phe Gly
   675                 680                 685                 690
   Lys Gly Ala Thr Ala Pro Val Lys Asp Ala Ala Asn Ala Asn Thr Leu
                   695                 700                 705
   Ala Thr Asn Tyr Ile Tyr Thr Phe Thr Thr Glu Gly Gln Asp Val Thr
               710                 715                 720
   Ala Pro Thr Val Thr Lys Val Phe Lys Gly Asp Ser Leu Lys Asp Ala
           725                 730                 735
   Asp Ala Val Thr Thr Leu Thr Asn Val Asp Ala Gly Gln Lys Phe Thr
       740                 745                 750
   Ile Gln Phe Ser Glu Glu Leu Lys Thr Ser Ser Gly Ser Leu Val Gly
   755                 760                 765                 770
   Gly Lys Val Thr Val Glu Lys Leu Thr Asn Asn Gly Trp Val Asp Ala
                   775                 780                 785
   Gly Thr Gly Thr Thr Val Ser Val Ala Pro Lys Thr Asp Ala Asn Gly
               790                 795                 800
   Lys Val Thr Ala Ala Val Val Thr Leu Thr Gly Leu Asp Asn Asn Asp
           805                 810                 815
   Lys Asp Ala Lys Leu Arg Leu Val Val Asp Lys Ser Ser Thr Asp Gly
       820                 825                 830
   Ile Ala Asp Val Ala Gly Asn Val Ile Lys Glu Lys Asp Ile Leu Ile
   835                 840                 845                 850
   Arg Tyr Asn Ser Trp Arg His Thr Val Ala Ser Val Lys Ala Ala Ala
                   855                 860                 865
   Asp Lys Asp Gly Gln Asn Ala Ser Ala Ala Phe Pro Thr Ser Thr Ala
               870                 875                 880
   Ile Asp Thr Thr Lys Ser Leu Leu Val Glu Phe Asn Glu Thr Asp Leu
           885                 890                 895
   Ala Glu Val Lys Pro Glu Asn Ile Val Val Lys Asp Ala Ala Gly Asn
       900                 905                 910
   Ala Val Ala Gly Thr Val Thr Ala Leu Asp Gly Ser Thr Asn Lys Phe
   915                 920                 925                 930
   Val Phe Thr Pro Ser Gln Glu Leu Lys Ala Gly Thr Val Tyr Ser Val
                   935                 940                 945
   Thr Ile Asp Gly Val Arg Asp Lys Val Gly Asn Thr Ile Ser Lys Tyr
               950                 955                 960
   Ile Thr Ser Phe Lys Thr Val Ser Ala Asn Pro Thr Leu Ser Ser Ile
           965                 970                 975
   Ser Ile Ala Asp Gly Ala Val Asn Val Asp Arg Ser Lys Thr Ile Thr
       980                 985                 990
   Ile Glu Phe Ser Asp Ser Val Pro Asn Pro Thr Ile Thr Leu Lys Lys
   995                 1000                1005                1010
   Ala Asp Gly Thr Ser Phe Thr Asn Tyr Thr Leu Val Asn Val Asn Asn
                   1015                1020                1025
   Glu Asn Lys Thr Tyr Lys Ile Val Phe His Lys Gly Val Thr Leu Asp
               1030                1035                1040
   Glu Phe Thr Gln Tyr Glu Leu Ala Val Ser Lys Asp Phe Gln Thr Gly
           1045                1050                1055
   Thr Asp Ile Asp Ser Lys Val Thr Phe Ile Thr Gly Ser Val Ala Thr
       1060                1065                1070
   Asp Glu Val Lys Pro Ala Leu Val Gly Val Gly Ser Trp Asn Gly Thr
   1075                1080                1085                1090
   Ser Tyr Thr Gln Asp Ala Ala Ala Thr Arg Leu Arg Ser Val Ala Asp
                   1095                1100                1105
   Phe Val Ala Glu Pro Val Ala Leu Gln Phe Ser Glu Gly Ile Asp Leu
               1110                1115                1120
   Thr Asn Ala Thr Val Thr Val Thr Asn Ile Thr Asp Asp Lys Thr Val
           1125                1130                1135
   Glu Val Ile Ser Lys Glu Ser Val Asp Ala Asp His Asp Ala Gly Ala
       1140                1145                1150
   Thr Lys Glu Thr Leu Val Ile Asn Thr Val Thr Pro Leu Val Leu Asp
   1155                1160                1165                1170
   Asn Ser Lys Thr Tyr Lys Ile Val Val Ser Gly Val Lys Asp Ala Ala
                   1175                1180                1185
   Gly Asn Val Ala Asp Thr Ile Thr Phe Tyr Ile Lys
               1190                1195
(2) ANGABEN ZU SEQ ID NO: 3:
   (i) SEQUENZKENNZEICHEN:
      (A) LÄNGE: 33 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 3:
      TTAATCGATT CTAGATGGAT AGGAAAAAAG CTG 33
(2) ANGABEN ZU SEQ ID NO: 4:
   (i) SEQUENZKENNZEICHEN:
      (A) LÄNGE: 37 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 4:
      ATACCCGGGG GTACGGATCC GATACAGATT TGAGCAA 37
(2) ANGABEN ZU SEQ ID NO: 5:
   (i) SEQUENZKENNZEICHEN:
      (A) LÄNGE: 2766 Basenpaare
      (B) ART: Nucleotid
      (C) STRANGFORM: beides
      (D) TOPOLOGIE: linear
   (vi) URSPRÜNGLICHE HERKUNFT:
      (A) ORGANISMUS: Bacillus stearothermophilus
      (B) STAMM: PV72
   (vii) UNMITTELBARE HERKUNFT:
      (B) CLON (E) : sbsB
   (ix) MERKMAL:
      (A) NAME/SCHLÜSSEL: CDS
      (B) LAGE:1..2763
   (ix) MERKMAL:
      (A) NAME/SCHLÜSSEL: sig_peptide
      (B) LAGE:1..93
   (ix) MERKMAL:
      (A) NAME/SCHLÜSSEL: mat_peptide
      (B) LAGE:94..2763
   (xi) SEQUENZBESCHREIBUNG: SEQ ID NO: 5:
   ATG GCT TAT CAA CCT AAG TCT TTT CGC AAG TTT GTT GCG ACA ACT GCA           48
   Met Ala Tyr Gln Pro Lys Ser Phe Arg Lys Phe Val Ala Thr Thr Ala
   -31 -30                 -25                     -20
   ACA GCT GCC ATT GTA GCA TCT GCG GTA GCT CCT GTA GTA TCT GCA GCA           96
   Thr Ala Ala Ile Val Ala Ser Ala Val Ala Pro Val Val Ser Ala Ala
   -15                 -10                     -5                  1
   AGC TTC ACA GAT GTT GCG CCG CAA TAT AAA GAT GCG ATC GAT TTC TTA          144
   Ser Phe Thr Asp Val Ala Pro Gln Tyr Lys Asp Ala Ile Asp Phe Leu
                   5                   10                  15
   GTA TCA ACT GGT GCA ACA AAA GGT AAA ACA GAA ACA AAA TTC GGC GTT          192
   Val Ser Thr Gly Ala Thr Lys Gly Lys Thr Glu Thr Lys Phe Gly Val
           20                  25                  30
   TAC GAT GAA ATC ACT CGT CTA GAT GCG GCA GTT ATT CTT GCA AGA GTA          240
   Tyr Asp Glu Ile Thr Arg Leu Asp Ala Ala Val Ile Leu Ala Arg Val
       35                  40                  45
   TTA AAA CTA GAC GTT GAC AAC GCA AAA GAC GCA GGC TTC ACA GAT GTG          288
   Leu Lys Leu Asp Val Asp Asn Ala Lys Asp Ala Gly Phe Thr Asp Val
    50                 55                  60                  65
   CCA AAA GAC CGT GCA AAA TAC GTC AAC GCG CTT GTA GAA GCT GGC GTA          336
   Pro Lys Asp Arg Ala Lys Tyr Val Asn Ala Leu Val Glu Ala Gly Val
                       70                  75                  80
   TTA AAC GGT AAA GCA CCT GGC AAA TTT GGT GCA TAC GAC CCA TTA ACT          384
   Leu Asn Gly Lys Ala Pro Gly Lys Phe Gly Ala Tyr Asp Pro Leu Thr
               85                  90                  95
   CGC GTT GAA ATG GCA AAA ATC ATC GCG AAC CGT TAC AAA TTA AAA GCT          432
   Arg Val Glu Met Ala Lys Ile Ile Ala Asn Arg Tyr Lys Leu Lys Ala
           100                 105                 110
   GAC GAT GTA AAA CTT CCA TTC ACT GAT GTA AAC GAT ACA TGG GCA CCA          480
   Asp Asp Val Lys Leu Pro Phe Thr Asp Val Asn Asp Thr Trp Ala Pro
       115                 120                 125
   TAC GTA AAA GCG CTT TAT AAA TAC GAA GTA ACC AAA AGG TTA AAA CAC          528
   Tyr Val Lys Ala Leu Tyr Lys Tyr Glu Val Thr Lys Arg Leu Lys His
   130                 135                 140                 145
   CAA CAA GCT TCG GTG CAT ACC AAA AAC ATC ACT CTG CGT GAC TTT GCG          576
   Gln Gln Ala Ser Val His Thr Lys Asn Ile Thr Leu Arg Asp Phe Ala
                   150                 155                 160
   CAA TTT GTA TAT AGA GCG GTG AAT ATT AAT GCA GTG CCA GAA ATA GTT          624
   Gln Phe Val Tyr Arg Ala Val Asn Ile Asn Ala Val Pro Glu Ile Val
               165                 170                 175
   GAA GTA ACT GCG GTT AAT TCG ACT ACA GTG AAA GTA ACA TTC AAT ACG        672
   Glu Val Thr Ala Val Asn Ser Thr Thr Val Lys Val Thr Phe Asn Thr
           180                 185                 190
   CAA ATT GCT GAT GTT GAT TTC ACA AAT TTT GCT ATC GAT AAC GGT TTA        720
   Gln Ile Ala Asp Val Asp Phe Thr Asn Phe Ala Ile Asp Asn Gly Leu
       195                 200                 205
   ACT GTT ACT AAA GCA ACT CTT TCT CGT GAT AAA AAA TCC GTA GAG GTT        768
   Thr Val Thr Lys Ala Thr Leu Ser Arg Asp Lys Lys Ser Val Glu Val
   210                 215                 220                 225
   GTG GTA AAT AAA CCG TTT ACT CGT AAT CAG GAA TAT ACA ATT ACA GCG        816
   Val Val Asn Lys Pro Phe Thr Arg Asn Gln Glu Tyr Thr Ile Thr Ala
                   230                 235                 240
   ACA GGC ATT AAA AAT TTA AAA GGC GAG ACC GCT AAG GAA TTA ACT GGT        864
   Thr Gly Ile Lys Asn Leu Lys Gly Glu Thr Ala Lys Glu Leu Thr Gly
               245                 250                 255
   AAG TTT GTT TGG TCT GTT CAA GAT GCG GTA ACT GTT GCA CTA AAT AAT        912
   Lys Phe Val Trp Ser Val Gln Asp Ala Val Thr Val Ala Leu Asn Asn
           260                 265                 270
   AGT TCG CTT AAA GTT GGA GAG GAA TCT GGT TTA ACT GTA AAA GAT CAG        960
   Ser Ser Leu Lys Val Gly Glu Glu Ser Gly Leu Thr Val Lys Asp Gln
       275                 280                 285
   GAT GGC AAA GAT GTT GTA GGT GCT AAA GTA GAA CTT ACT TCT TCT AAT       1008
   Asp Gly Lys Asp Val Val Gly Ala Lys Val Glu Leu Thr Ser Ser Asn
   290                 295                 300                 305
   ACT AAT ATT GTT GTA GTT TCA AGT GGC GAA GTA TCA GTA TCT GCT GCT       1056
   Thr Asn Ile Val Val Val Ser Ser Gly Glu Val Ser Val Ser Ala Ala
                   310                 315                 320
   AAA GTT ACA GCT GTA AAA CCG GGA ACA GCT GAT GTT ACT GCA AAA GTT       1104
   Lys Val Thr Ala Val Lys Pro Gly Thr Ala Asp Val Thr Ala Lys Val
               325                 330                 335
   ACA TTA CCA GAT GGT GTT GTA CTA ACA AAT ACA TTT AAA GTG ACA GTT       1152
   Thr Leu Pro Asp Gly Val Val Leu Thr Asn Thr Phe Lys Val Thr Val
           340                 345                 350
   ACA GAA GTG CCT GTT CAA GTC CAA AAT CAA GGA TTT ACT TTA GTT GAT       1200
   Thr Glu Val Pro Val Gln Val Gln Asn Gln Gly Phe Thr Leu Val Asp
       355                 360                 365
   AAT CTT TCT AAT GCT CCA CAG AAT ACA GTT GCA TTT AAC AAA GCT GAG       1248
   Asn Leu Ser Asn Ala Pro Gln Asn Thr Val Ala Phe Asn Lys Ala Glu
   370                 375                 380                 385
   AAA GTA ACT TCA ATG TTT GCT GGA GAA ACT AAA ACA GTT GCA ATG TAT       1296
   Lys Val Thr Ser Met Phe Ala Gly Glu Thr Lys Thr Val Ala Met Tyr
                   390                 395                 400
   GAT ACT AAA AAC GGT GAT CCT GAA ACT AAA CCT GTT GAT TTC AAA GAT       1344
   Asp Thr Lys Asn Gly Asp Pro Glu Thr Lys Pro Val Asp Phe Lys Asp
               405                 410                 415
   GCA ACT GTA CGT TCA TTA AAT CCA ATT ATT GCA ACA GCT GCT ATT AAT       1392
   Ala Thr Val Arg Ser Leu Asn Pro Ile Ile Ala Thr Ala Ala Ile Asn
           420                 425                 430
   GGT AGT GAG CTC CTT GTC ACA GCT AAT GCT GGC CAA TCT GGA AAA GCT       1440
   Gly Ser Glu Leu Leu Val Thr Ala Asn Ala Gly Gln Ser Gly Lys Ala
       435                 440                 445
   TCA TTT GAA GTA ACA TTA AAA GAT AAT ACA AAA AGA ACA TTT ACA GTT       1488
   Ser Phe Glu Val Thr Leu Lys Asp Asn Thr Lys Arg Thr Phe Thr Val
   450                 455                 460                 465
   GAT GTA AAA AAA GAC CCT GTA TTA CAA GAT ATA AAA GTA GAT GCA ACT      1536
   Asp Val Lys Lys Asp Pro Val Leu Gln Asp Ile Lys Val Asp Ala Thr
                   470                 475                 480
   TCT GTT AAA CTT TCC GAT GAA GCT GTT GGC GGC GGG GAA GTT GAA GGA      1584
   Ser Val Lys Leu Ser Asp Glu Ala Val Gly Gly Gly Glu Val Glu Gly
               485                 490                 495
   GTT AAC CAA AAA ACG ATT AAA GTA AGT GCA GTT GAC CAA TAC GGT AAA      1632
   Val Asn Gln Lys Thr Ile Lys Val Ser Ala Val Asp Gln Tyr Gly Lys
           500                 505                 510
   GAA ATT AAA TTT GGT ACA AAA GGT AAA GTT ACT GTT ACA ACT AAT ACA      1680
   Glu Ile Lys Phe Gly Thr Lys Gly Lys Val Thr Val Thr Thr Asn Thr
       515                 520                 525
   GAA GGA CTA GTT ATT AAA AAT GTA AAT AGC GAT AAT ACA ATT GAC TTT      1728
   Glu Gly Leu Val Ile Lys Asn Val Asn Ser Asp Asn Thr Ile Asp Phe
   530                 535                 540                 545
   GAT AGC GGC AAT AGT GCA ACT GAC CAA TTT GTT GTC GTT GCA ACA AAA      1776
   Asp Ser Gly Asn Ser Ala Thr Asp Gln Phe Val Val Val Ala Thr Lys
                   550                 555                 560
   GAC AAA ATT GTC AAT GGT AAA GTA GAA GTT AAA TAT TTC AAA AAT GCT      1824
   Asp Lys Ile Val Asn Gly Lys Val Glu Val Lys Tyr Phe Lys Asn Ala
               565                 570                 575
   AGT GAC ACA ACA CCA ACT TCA ACT AAA ACA ATT ACT GTT AAT GTA GTA      1872
   Ser Asp Thr Thr Pro Thr Ser Thr Lys Thr Ile Thr Val Asn Val Val
           580                 585                 590
   AAT GTA AAA GCT GAC GCT ACA CCA GTA GGA TTA GAT ATT GTA GCA CCT      1920
   Asn Val Lys Ala Asp Ala Thr Pro Val Gly Leu Asp Ile Val Ala Pro
       595                 600                 605
   TCT AAA ATT GAT GTA AAT GCT CCA AAC ACT GCT TCT ACT GCA GAT GTT      1968
   Ser Lys Ile Asp Val Asn Ala Pro Asn Thr Ala Ser Thr Ala Asp Val
   610                 615                 620                 625
   GAT TTT ATA AAT TTC GAA AGT GTT GAG ATT TAC ACA CTC GAT TCA AAT      2016
   Asp Phe Ile Asn Phe Glu Ser Val Glu Ile Tyr Thr Leu Asp Ser Asn
                   630                 635                 640
   GGT AGA CGT CAA AAA AAA GTT ACT CCA ACT GCA ACT ACA CTT GTA GGT      2064
   Gly Arg Arg Gln Lys Lys Val Thr Pro Thr Ala Thr Thr Leu Val Gly
               645                 650                 655
   ACA AAA AAA AAA AAA AAA GTT AAT GGG AAT GTA TTA CAA TTC AAG GGG      2112
   Thr Lys Lys Lys Lys Lys Val Asn Gly Asn Val Leu Gln Phe Lys Gly
           660                 665                 670
   AAC GAA GAA TTA ACG CTA TCA ACT TCT TCT AGT ACA GGA AAC GTA GAT      2160
   Asn Glu Glu Leu Thr Leu Ser Thr Ser Ser Ser Thr Gly Asn Val Asp
       675                 680                 685
   GGA ACA GCA GAA GGA ATG ACA AAA CGT ATT CCA GGG AAA TAT ATC AAC      2208
   Gly Thr Ala Glu Gly Met Thr Lys Arg Ile Pro Gly Lys Tyr Ile Asn
   690                 695                 700                 705
   TCT GCA AGT GTA CCT GCC AGT GCA ACA GTA GCA ACA AGT CCT GTT ACT      2256
   Ser Ala Ser Val Pro Ala Ser Ala Thr Val Ala Thr Ser Pro Val Thr
                   710                 715                 720
   GTA AAG CTT AAT TCA AGT GAT AAT GAT TTA ACA TTT GAA GAA TTA ATA      2304
   Val Lys Leu Asn Ser Ser Asp Asn Asp Leu Thr Phe Glu Glu Leu Ile
               725                 730                 735
   TTC GGT GTA ATT GAC CCT ACA CAA TTA GTC AAA GAT GAA GAC ATC AAC      2352
   Phe Gly Val Ile Asp Pro Thr Gln Leu Val Lys Asp Glu Asp Ile Asn
           740                 745                 750
   GAA TTT ATT GCA GTT TCA AAA GCG GCT AAA AAT GAT GGA TAT TTG TAT      2400
   Glu Phe Ile Ala Val Ser Lys Ala Ala Lys Asn Asp Gly Tyr Leu Tyr
       755                 760                 765
   AAT AAA CCG CTT GTA ACG GTT AAA GAT GCA TCA GGA AAA GTT ATT CCA      2448
   Asn Lys Pro Leu Val Thr Val Lys Asp Ala Ser Gly Lys Val Ile Pro
   770                 775                 780                 785
   ACA GGT GCA AAT GTT TAC GGT CTA AAT CAT GAT GCA ACT AAC GGA AAC      2496
   Thr Gly Ala Asn Val Tyr Gly Leu Asn His Asp Ala Thr Asn Gly Asn
                   790                 795                 800
   ATT TGG TTT GAT GAG GAA CAA GCT GGC TTA GCT AAA AAA TTT AGT GAT      2544
   Ile Trp Phe Asp Glu Glu Gln Ala Gly Leu Ala Lys Lys Phe Ser Asp
               805                 810                 815
   GTA CAT TTT GAT GTT GAT TTT TCA TTA ACT AAC GTT GTA AAA ACT GGT      2592
   Val His Phe Asp Val Asp Phe Ser Leu Thr Asn Val Val Lys Thr Gly
           820                 825                 830
   AGC GGT ACA GTT TCT TCA TCG CCA TCA TTA TCT GAC GCA ATT CAA CTT      2640
   Ser Gly Thr Val Ser Ser Ser Pro Ser Leu Ser Asp Ala Ile Gln Leu
       835                 840                 845
   ACT AAT TCA GGC GAT GCA GTA TCG TTT ACA TTA GTT ATC AAA TCA ATT      2688
   Thr Asn Ser Gly Asp Ala Val Ser Phe Thr Leu Val Ile Lys Ser Ile
   850                 855                 860                 865
   TAT GTT AAA GGC GCA GAT AAA GAT GAT AAT AAC TTA CTT GCA GCC CCT      2736
   Tyr Val Lys Gly Ala Asp Lys Asp Asp Asn Asn Leu Leu Ala Ala Pro
                   870                 875                 880
   GTT TCT GTC AAT GTG ACT GTG ACA AAA TAA                              2766
   Val Ser Val Asn Val Thr Val Thr Lys
               885                 890
(2) ANGABEN ZU SEQ ID NO: 6:
   (i) SEQUENZKENNZEICHEN:
      (A) LÄNGE: 921 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 6:
   Met Ala Tyr Gln Pro Lys Ser Phe Arg Lys Phe Val Ala Thr Thr Ala
   -31 -30                 -25                 -20
   Thr Ala Ala Ile Val Ala Ser Ala Val Ala Pro Val Val Ser Ala Ala
   -15                 -10                 -5                      1
   Ser Phe Thr Asp Val Ala Pro Gln Tyr Lys Asp Ala Ile Asp Phe Leu
                   5                   10                  15
   Val Ser Thr Gly Ala Thr Lys Gly Lys Thr Glu Thr Lys Phe Gly Val
               20                  25                  30
   Tyr Asp Glu Ile Thr Arg Leu Asp Ala Ala Val Ile Leu Ala Arg Val
           35                  40                  45
   Leu Lys Leu Asp Val Asp Asn Ala Lys Asp Ala Gly Phe Thr Asp Val
       50                  55                  60                  65
   Pro Lys Asp Arg Ala Lys Tyr Val Asn Ala Leu Val Glu Ala Gly Val
                       70                  75                  80
   Leu Asn Gly Lys Ala Pro Gly Lys Phe Gly Ala Tyr Asp Pro Leu Thr
                   85                  90                  95
   Arg Val Glu Met Ala Lys Ile Ile Ala Asn Arg Tyr Lys Leu Lys Ala
               100                 105                 110
   Asp Asp Val Lys Leu Pro Phe Thr Asp Val Asn Asp Thr Trp Ala Pro
           115                 120                 125
   Tyr Val Lys Ala Leu Tyr Lys Tyr Glu Val Thr Lys Arg Leu Lys His
       130                 135                 140                 145
   Gln Gln Ala Ser Val His Thr Lys Asn Ile Thr Leu Arg Asp Phe Ala
                       150                 155                 160
   Gln Phe Val Tyr Arg Ala Val Asn Ile Asn Ala Val Pro Glu Ile Val
                   165                 170                 175
   Glu Val Thr Ala Val Asn Ser Thr Thr Val Lys Val Thr Phe Asn Thr
               180                 185                 190
   Gln Ile Ala Asp Val Asp Phe Thr Asn Phe Ala Ile Asp Asn Gly Leu
           195                 200                 205
   Thr Val Thr Lys Ala Thr Leu Ser Arg Asp Lys Lys Ser Val Glu Val
       210                 215                 220                 225
   Val Val Asn Lys Pro Phe Thr Arg Asn Gln Glu Tyr Thr Ile Thr Ala
                       230                 235                 240
   Thr Gly Ile Lys Asn Leu Lys Gly Glu Thr Ala Lys Glu Leu Thr Gly
                   245                 250                 255
   Lys Phe Val Trp Ser Val Gln Asp Ala Val Thr Val Ala Leu Asn Asn
               260                 265                 270
   Ser Ser Leu Lys Val Gly Glu Glu Ser Gly Leu Thr Val Lys Asp Gln
           275                 280                 285
   Asp Gly Lys Asp Val Val Gly Ala Lys Val Glu Leu Thr Ser Ser Asn
       290                 295                 300                 305
   Thr Asn Ile Val Val Val Ser Ser Gly Glu Val Ser Val Ser Ala Ala
                       310                 315                 320
   Lys Val Thr Ala Val Lys Pro Gly Thr Ala Asp Val Thr Ala Lys Val
                   325                 330                 335
   Thr Leu Pro Asp Gly Val Val Leu Thr Asn Thr Phe Lys Val Thr Val
               340                 345                 350
   Thr Glu Val Pro Val Gln Val Gln Asn Gln Gly Phe Thr Leu Val Asp
           355                 360                 365
   Asn Leu Ser Asn Ala Pro Gln Asn Thr Val Ala Phe Asn Lys Ala Glu
       370                 375                 380                 385
   Lys Val Thr Ser Met Phe Ala Gly Glu Thr Lys Thr Val Ala Met Tyr
                       390                 395                 400
   Asp Thr Lys Asn Gly Asp Pro Glu Thr Lys Pro Val Asp Phe Lys Asp
                   405                 410                 415
   Ala Thr Val Arg Ser Leu Asn Pro Ile Ile Ala Thr Ala Ala Ile Asn
               420                 425                 430
   Gly Ser Glu Leu Leu Val Thr Ala Asn Ala Gly Gln Ser Gly Lys Ala
           435                 440                 445
   Ser Phe Glu Val Thr Leu Lys Asp Asn Thr Lys Arg Thr Phe Thr Val
       450                 455                 460                 465
   Asp Val Lys Lys Asp Pro Val Leu Gln Asp Ile Lys Val Asp Ala Thr
                   470                 475                 480
   Ser Val Lys Leu Ser Asp Glu Ala Val Gly Gly Gly Glu Val Glu Gly
               485                 490                 495
   Val Asn Gln Lys Thr Ile Lys Val Ser Ala Val Asp Gln Tyr Gly Lys
           500                 505                 510
   Glu Ile Lys Phe Gly Thr Lys Gly Lys Val Thr Val Thr Thr Asn Thr
       515                 520                 525
   Glu Gly Leu Val Ile Lys Asn Val Asn Ser Asp Asn Thr Ile Asp Phe
   530                 535                 540                 545
   Asp Ser Gly Asn Ser Ala Thr Asp Gln Phe Val Val Val Ala Thr Lys
                   550                 555                 560
   Asp Lys Ile Val Asn Gly Lys Val Glu Val Lys Tyr Phe Lys Asn Ala
               565                 570                 575
   Ser Asp Thr Thr Pro Thr Ser Thr Lys Thr Ile Thr Val Asn Val Val
           580                 585                 590
   Asn Val Lys Ala Asp Ala Thr Pro Val Gly Leu Asp Ile Val Ala Pro
       595                 600                 605
   Ser Lys Ile Asp Val Asn Ala Pro Asn Thr Ala Ser Thr Ala Asp Val
   610                 615                 620                 625
   Asp Phe Ile Asn Phe Glu Ser Val Glu Ile Tyr Thr Leu Asp Ser Asn
                   630                 635                 640
   Gly Arg Arg Gln Lys Lys Val Thr Pro Thr Ala Thr Thr Leu Val Gly
               645                 650                 655
   Thr Lys Lys Lys Lys Lys Val Asn Gly Asn Val Leu Gln Phe Lys Gly
           660                 665                 670
   Asn Glu Glu Leu Thr Leu Ser Thr Ser Ser Ser Thr Gly Asn Val Asp
       675                 680                 685
   Gly Thr Ala Glu Gly Met Thr Lys Arg Ile Pro Gly Lys Tyr Ile Asn
   690                 695                 700                 705
   Ser Ala Ser Val Pro Ala Ser Ala Thr Val Ala Thr Ser Pro Val Thr
                   710                 715                 720
   Val Lys Leu Asn Ser Ser Asp Asn Asp Leu Thr Phe Glu Glu Leu Ile
               725                 730                 735
   Phe Gly Val Ile Asp Pro Thr Gln Leu Val Lys Asp Glu Asp Ile Asn
           740                 745                 750
   Glu Phe Ile Ala Val Ser Lys Ala Ala Lys Asn Asp Gly Tyr Leu Tyr
       755                 760                 765
   Asn Lys Pro Leu Val Thr Val Lys Asp Ala Ser Gly Lys Val Ile Pro
   770                 775                 780                 785
   Thr Gly Ala Asn Val Tyr Gly Leu Asn His Asp Ala Thr Asn Gly Asn
                   790                 795                 800
   Ile Trp Phe Asp Glu Glu Gln Ala Gly Leu Ala Lys Lys Phe Ser Asp
               805                 810                 815
   Val His Phe Asp Val Asp Phe Ser Leu Thr Asn Val Val Lys Thr Gly
           820                 825                 830
   Ser Gly Thr Val Ser Ser Ser Pro Ser Leu Ser Asp Ala Ile Gln Leu
       835                 840                 845
   Thr Asn Ser Gly Asp Ala Val Ser Phe Thr Leu Val Ile Lys Ser Ile
   850                 855                 860                 865
   Tyr Val Lys Gly Ala Asp Lys Asp Asp Asn Asn Leu Leu Ala Ala Pro
                   870                 875                 880
   Val Ser Val Asn Val Thr Val Thr Lys
               885                 890
(2) ANGABEN ZU SEQ ID NO: 7:
   (i) SEQUENZKENNZEICHEN:
      (A) LÄNGE: 75 Basenpaare
      (B) ART: Nucleotid
      (C) STRANGFORM: beides
      (D) TOPOLOGIE: linear
   (xi) SEQUENZBESCHREIBUNG: SEQ ID NO: 7:
   ATGAAAATAA AAACAGGTGC ACGCATCCTC GCATTATCCG CATTAACGAC
   GATGATGTTT TCCGCCTCGG CTCTC
(2) ANGABEN ZU SEQ ID NO: 8:
   (i) SEQUENZKENNZEICHEN:
      (A) LÄNGE: 45 Basenpaare
      (B) ART: Nucleotid
      (C) STRANGFORM: beides
      (D) TOPOLOGIE: linear
   (xi) SEQUENZBESCHREIBUNG: SEQ ID NO: 8:
   GTGAAAAAAT TATTATTCGC AATTCCTTTA
   GTTGTTCCTT TCTAT